(19) Europäisches Patentamt
European Patent Office
Office européen des brevets

(11) EP 1 996 166 B1

(12) # FASCICULE DE BREVET EUROPEEN

(45) Date de publication et mention de la délivrance du brevet:
**05.10.2016 Bulletin 2016/40**

(21) Numéro de dépôt: **07712415.4**

(22) Date de dépôt: **01.03.2007**

(51) Int Cl.:
***A61K 31/485*** *(2006.01)* ***A61K 9/22*** *(2006.01)*

(86) Numéro de dépôt international:
**PCT/EP2007/051967**

(87) Numéro de publication internationale:
**WO 2007/099152 (07.09.2007 Gazette 2007/36)**

(54) **COMPRIMÉS RÉSISTANT À L'ÉCRASEMENT DESTINÉS À ÉVITER LE MÉSUSAGE INVOLONTAIRE ET LE DÉTOURNEMENT ILLICITE**

GEGEN ZERDRÜCKEN WIDERSTANDSFÄHIGE TABLETTEN ZUR PRÄVENTION VON VERSEHENTLICHEM MISSBRAUCH UND ILLEGALER NUTZUNG

CRUSH-RESISTANT TABLETS INTENDED TO PREVENT ACCIDENTAL MISUSE AND UNLAWFUL DIVERSION

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(30) Priorité: **01.03.2006 FR 0601842**

(43) Date de publication de la demande:
**03.12.2008 Bulletin 2008/49**

(60) Demande divisionnaire:
**16183449.4**

(73) Titulaire: **Ethypharm**
**92210 Saint-Cloud (FR)**

(72) Inventeurs:
- **CAILLY-DUFESTEL, Vincent**
**F-76710 Bosc-Guérard-saint-Adrien (FR)**
- **HERRY, Catherine**
**F-76320 Saint Pierre lès Elbeuf (FR)**
- **BACON, Jonathan**
**Montreal, H1Y 2Z3 (CA)**
- **OURY, Pascal**
**F-78150 Le Chesnay (FR)**

(74) Mandataire: **Regimbeau**
**20, rue de Chazelles**
**75847 Paris Cedex 17 (FR)**

(56) Documents cités:
**US-A1- 2005 031 546**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention concerne des comprimés matriciels insolubles présentant une très forte résistance à l'écrasement.

Ces comprimés matriciels incassables dans les conditions habituelles, non friables et insolubles en milieu aqueux sont particulièrement intéressants comme réservoirs d'agents psychotropes puisqu'ils permettent de réduire, voire d'empêcher, les utilisations détournées de ces produits à des fins toxicomanes, par broyage, dissolution et injection ou par broyage et inhalation.

La présente invention concerne en outre le procédé d'obtention de tels comprimés ainsi que leur utilisation pour l'administration orale prolongée de principes actifs et en particulier de principes actifs psychotropes.

Dans le cas de comprimés d'agents opiacés à libération prolongée, et en particulier de l'oxycodone, le phénomène de mésusage involontaire peut revêtir plusieurs aspects. Tout d'abord, il peut être le fait d'un non respect des conditions d'administration. En effet, il arrive que le comprimé destiné à être avalé soit mâché accidentellement par le patient. Les conséquences d'une destruction partielle ou totale du comprimé dont la structure est destinée à ralentir la libération du principe actif peuvent s'avérer dangereuses voire fatales pour le patient (surdosage pouvant conduire à une overdose). C'est pourquoi, par exemple, la notice du médicament Oxycontin® LP précise bien que *« Les comprimés doivent être avalés entiers sans être croqués ».*

Par ailleurs, le mésusage non intentionnel de médicaments à base d'oxycodone à libération prolongée a également été observé lorsque le patient ingère simultanément ou de façon rapprochée dans le temps le médicament avec une forte dose d'alcool.

En effet, il a été observé sur une forme retard d'hydromorphone que la présence d'alcool dans l'estomac altérait la couche d'excipients responsables de la libération prolongée du principe actif, conduisant au relargage dans l'organisme d'une quantité importante d'actif *(«dose-dumping"),* responsable, là encore d'un surdosage dangereux.
Ainsi, la notice de l'Oxycontin® LP mentionne par exemple, dans la liste des contre-indications que l'ingestion d'alcool est à proscrire avec ce médicament. De même, aux Etats-Unis, la FDA *(Food and Drug Administration)* met sérieusement en garde les patients traités par l'Oxycontin® contre l'absorption de boissons alcoolisées pendant leur traitement (voir notamment : http://www.fda.gov/cder/drug/infopage/oxycontin/oxycontin -qa.htm).

Il existe donc un réel besoin pour prévenir ce type de mésusage accidentel pour augmenter la sécurité du patient, tout en conservant une voie d'administration simple et confortable (voie orale).

Depuis l'apparition sur le marché pharmaceutique français en 1990 de traitements de substitution aux drogues opiacées sous forme de comprimés sublinguaux (Temgésic®) initialement conditionnés sous forme injectable, on a constaté une amplification du phénomène de détournement de certains actifs psychotropes par la population toxicomane.

On parle de détournement ou de mésusage volontaire (ou encore, suivant le terme anglo-saxon de "*drug-abuse*") pour qualifier l'utilisation détournée à des fins toxicomanes de certains médicaments, en particulier de certains agents psychotropes ou stupéfiants, tels que les opioïdes ou leurs dérivés utilisés en thérapeutique dans le traitement des douleurs sévères ou dans le traitement de l'addiction aux drogues opiacées.

Le détournement par voie parentérale/pernasale d'actifs à effet retard normalement destinés à la voie orale permet au toxicomane de ressentir immédiatement après administration les effets psychotropes cumulés de la dose totale d'actif présente dans la formulation initiale.

Ainsi, dans le cas particulier de la buprenorphine, antalgique opioïde puissant initialement vendu sous le nom de spécialité Temgésic® comme traitement de substitution destiné aux toxicomanes, on estime que 25 % à 30% des traitements vendus sont détournés pour un usage par voie parentérale ou nasale. Il en va de même pour la spécialité Subutex® (comprimés de buprénorphine sublinguale à haut dosage de Schering-Plough) utilisée officiellement comme traitement de substitution chez plusieurs dizaines de milliers de toxicomanes aux opiacés, pour laquelle on estime que 34% des consommateurs la détournent en se l'administrant par voie injectable et environ 30% par voie nasale.

Or, le phénomène de mésusage volontaire s'observe également pour des spécialités destinées au traitement des douleurs sévères, telles que le sulfate de morphine (Skenan®) et l'oxycodone par exemple (Moscontin®, Oxycontin® LP) ou des douleurs modérées (Neocodion®). Ces formes à libération prolongée contiennent des quantités importantes d'opioïdes destinées à limiter les douleurs pendant des durées longues et le détournement provoque alors la mise à disposition massive d'une quantité élevée de dérivés morphiniques.

Le détournement touche également d'autres classes thérapeutiques en particulier les benzodiazépines (Rohypnol®) et à moindre échelle, certains traitements neurologiques (Artane® médiament anti-parkinsonien).

Ainsi, ces traitements thérapeutiques ou de substitution, parfois accessibles sur simple ordonnance et dont la posologie peut atteindre une dizaine de comprimés par jour, sont sujets à deux modes principaux de détournement: une administration parentérale (injection) et une administration pernasale (inhalation).

Dans le cas d'un détournement par injection le comprimé ou la gélule contenant les principes actifs d'intérêt est réduit en poudre fine à l'aide de tous les moyens possibles à la disposition du toxicomane notamment à l'aide d'un mortier ou d'un briquet voire par simple mastication ou en croquant le comprimé. La poudre grossière obtenue, qui contient nécessairement les excipients initialement présents dans la forme galénique peut alors être dissoute dans un faible volume de liquide (quelques millilitres) parfois préalablement chauffé et/ou additionné d'acide dans le cas de certains actifs présents sous leur forme base (héroïne brune, morphine base). Le liquide obtenu peut ensuite être filtré sommairement, pour limiter l'introduction dans le sang de grosses particules, à l'aide d'un filtre à cigarette par exemple avant d'être injecté par voie intraveineuse.

Dans ce cas, le principe actif est alors immédiatement disponible dans le sang, puisque plus aucun excipient ne peut retarder sa libération, procurant l'effet psychotrope immédiat recherché par le toxicomane.

Le détournement par inhalation consiste également à broyer la forme galénique jusqu'à l'obtention d'une poudre suffisamment fine pour rendre le principe actif accessible aux micro-vaisseaux de la muqueuse intranasale. Là encore, l'action des excipients retards prévus pour une administration orale est tout à fait inefficace et l'effet psychotrope immédiat attendu peut se produire.

Le détournement s'accompagne en outre de nombreux risques sanitaires liés directement à l'injection ou à l'inhalation des excipients et des résidus de broyage non purifiés, peu ou mal filtrés et non stériles. En effet, des études récentes rapportent que certains comprimés détournés sont parfois dissous directement dans la seringue, puis injectés, sans aucune filtration préalable, cette pratique étant directement responsable de nombreux décès par embolie pulmonaire. De plus, l'adjonction d'acides sous des formes liquides non stériles (jus de citron) aux résidus de broyage serait également responsable de la transmission de pathologies bactériennes ou mycosiques (candidoses)

Ces pratiques viennent ainsi augmenter les risques déjà élevés de transmissions virales et bactériennes et de complications d'ordre dermatologique (abcès, nécroses) liées à l'injection parentérale proprement dite. Par ailleurs, dans le cas de l'injection de comprimés de Subutex®, la présence d'amidon de maïs dans la formule des comprimés est responsable de l'apparition d'oedèmes dus à cet excipient qui, une fois injecté, s'accumule dans les réseaux lymphatiques et veineux, ce qui entraîne un gonflement des membres inférieurs.

Pour limiter ces problèmes, une approche consiste à associer le principe actif dans une même forme galénique qu'un agent capable d'en limiter l'effet psychotrope lorsque la formulation est prise par voie parentérale.

C'est le cas par exemple des formulations combinant la méthadone et la naloxone initialement décrites dans les brevets US 3,966,940 et US 3,773,955.

Ce concept anti-détournement a été repris dans le cas particulier de la buprénorphine. Ainsi, le brevet EP 0 185 472, décrit une formulation orale de buprénorphine contenant également une dose efficace de naloxone, qui agit comme antagoniste compétitif au niveau des récepteurs morphiniques. La naloxone étant très peu biodisponible par voie orale, elle entrave peu l'action analgésique de la buprénorphine lorsque le médicament est administré de façon conventionnelle per os. En revanche, lorsqu'elle est soumise au détournement par voie parentérale, la naloxone est alors entièrement disponible et inhibe l'action analgésique de la buprénorphine. Cependant, dans ce type d'associations chimiques, la formulation galénique orale reste broyable et soluble en milieu aqueux.

Une formulation sublinguale associant naltrexone et buprénorphine a par ailleurs été décrite dans le brevet EP 0 319 243. Une telle association permet notamment d'augmenter l'effet antagoniste de la naltrexone vis à vis des opiacés, tout en procurant au consommateur une sensation analgésique non euphorisante même si la composition est détournée par voie parentérale. Ce genre de formulation est donc peu attractif pour le toxicomane et permet de freiner le phénomène de mésusage volontaire. Cependant, cette approche passe nécessairement par la co-administration de deux principes actifs, entraînant une augmentation du coût de production et du prix de vente du médicament.

Toujours dans une approche combinant l'association de l'opioïde avec un agent antagoniste, la demande de brevet US 2003/0143269 décrit une forme galénique dans laquelle l'opioïde et l'antagoniste sont interdispersés de telle sorte que l'antagoniste soit "séquestré" dans un compartiment qui l'empêche de se libérer lorsque le médicament est pris de façon normale, par voie orale. En revanche, en cas de tentative de détournement par broyage, l'altération de la structure entraîne le mélange des deux agents actifs, conduisant à une inhibition de l'effet psychotrope recherché.

Dans cette approche, la forme galénique joue un rôle prépondérant face au détournement, cependant, là encore, l'association chimique de deux composés est nécessaire, entraînant un procédé de fabrication complexe et un coût de production élevé.

Par ailleurs la demande de brevet US 2003/0068392 décrit une forme pharmaceutique dans laquelle l'agent opioïde est associé non seulement à un antagoniste, mais également à un agent irritant séquestré dans un compartiment clos. L'altération volontaire de la forme pharmaceutique entraîne inévitablement la libération de l'agent irritant. Cette forme nécessite donc l'association de trois agents actifs, et la création de zones compartimentées, ce qui rend sa fabrication complexe et plus coûteuse qu'une forme galénique simple comme un comprimé.

D'autres sociétés ont mis au point des systèmes galéniques dans lesquels l'opioïde ou la substance sujette au détournement n'est pas associée à un antagoniste. Ainsi, la demande de brevet US 2005/0281748 enseigne la fabrication d'une forme pharmaceutique orale dans laquelle l'agent opioïde d'intérêt est modifié de façon à augmenter sa lipophilie, en formant un sel entre l'actif et un ou plusieurs acides gras.

Cette forme galénique permet la délivrance prolongée de l'actif lorsque celui-ci est pris par voie orale, puisque les enzymes du suc digestif dégradent progressivement les groupements d'acide gras, libérant l'actif au fur et à mesure de leur dégradation.

En revanche, une altération physique de la forme galénique libère des microparticules d'actif enrobées d'une couche insoluble empêchant la libération immédiate de l'actif en milieu aqueux. Une telle formulation nécessite une transformation chimique du principe actif.

La demande de brevet US 2003/0118641 décrit une forme orale d'opioïde à libération prolongée dans laquelle l'agent actif opioïde est associé à une matrice polymérique hydrophile et une résine cationique. La résine portant des charges opposées à celle de l'agent actif elle se lie à ce dernier au sein de la matrice polymérique, empêchant son extraction.

Un tel support galénique rend le composé actif non séparable des excipients responsables de sa libération prolongée dans l'organisme, même à l'aide des principaux solvants commercialement disponibles (eau chaude, alcool, vinaigre, eau oxygénée, etc....).

Certaines sociétés ont mis au point des systèmes galéniques à base de gels. Ainsi, les sociétés Pain Therapeutics Inc. et Durect utilisent un gel biodégradable administrable par voie orale ou parentérale constitué d'un agent de haute viscosité, l'acétate d'isobutyrate de saccharose *(Sucrose Acetate Iso Butyrate* ou SAIB). Ce gel permet une libération prolongée dans le temps d'un agent opioïde, l'oxycodone. Ce type de gel, qui fait l'objet des brevets US 5,747,058 et US 6,413,536 conserve sa capacité à libérer le principe actif de façon contrôlée sur des périodes de 12 à 24 heures, même lorsque les capsules qui les contiennent sont altérées ou soumises au broyage. Le principal intérêt de ces formes galéniques tient au fait que l'oxycodone ne peut être extraite de son support gélifié, et ne peut pas non plus être injectée par voie parentérale du fait de la viscosité trop élevée de ces formulations (Produit Remoxy® utilisant les technologies ORADUR® et SABER® actuellement en phase III d'essais cliniques).

De tels gels présentent en outre la capacité de résister à une extraction de l'oxycodone en présence d'alcool ou d'acide, l'actif restant piégé dans le réseau formé par l'agent gélifiant.

Ces formes galéniques contenant des gels, sont des formulations complexes, qui d'une part nécessitent de travailler à l'échelle industrielle avec des liquides de haute viscosité, ce qui rend leur manipulation contraignante et d'autre part comportent des contraintes importantes sur le plan du conditionnement (utilisation de flacons et ou d'ampoules), ce qui n'est pas le cas avec des comprimés.

On connaît par ailleurs le moyen de fabriquer des comprimés matriciels présentant une très forte dureté. Ainsi, le brevet EP 0 974 355 décrit des comprimés obtenus par granulation d'une vitamine hydrosoluble en mélange avec au moins un additif de nature saccharidique en présence d'un liant polymérique classique comme l'HPMC par exemple. De tels comprimés, destinés à une libération rapide de la vitamine hydrosoluble dans l'organisme, présentent une dureté élevée, de l'ordre de 20 à 30 kp/cm$^2$ (kiloponds/cm$^2$), ce qui équivaut à des valeurs de dureté d'environ 1,96 à 2,94 MPa. Bien que relativement durs, ces comprimés constitués à plus de 90 % par une vitamine hydrosoluble et des excipients également hydrosolubles (HPMC, saccharides) sont rapidement dégradés dans l'organisme (temps de désintégration de l'ordre de 10 à 15 minutes). De tels comprimés sont d'une part totalement inadaptés à une libération prolongée du principe actif, mais par ailleurs facilement dissous en milieu aqueux, ce qui les rend impropres à servir de support galénique pour des substances sujettes au détournement.

Le brevet EP 0 933 079 décrit des comprimés matriciels présentant une résistance à l'écrasement qui varie d'environ 1 MPa (1 N/mm$^2$) jusqu'à 10 MPa. De tels comprimés sont obtenus à partir d'une poudre d'amidon traité, directement compressible. Cependant, ces comprimés sont destinés à la mise à disposition rapide de principes actifs, en effet, ils présentent un temps de désintégration en milieu aqueux relativement court, de l'ordre de 6 à 7 minutes environ. Du fait de leur rapide dissolution en milieu aqueux, ces comprimés sont, là encore, non utilisables pour le transport d'actifs sujets au mésusage volontaire et devant être libérés sur de longues périodes de temps.

Le brevet EP 0 997 143 décrit la fabrication de comprimés matriciels biconvexes de très grande dureté (jusqu'à 1,1 MPa soit environ 11 kp/cm$^2$) et d'une friabilité inférieure à 1 %, obtenus après compression d'une matrice constituée majoritairement par un agent carbohydrate compressible et désintégrant (en général le mannitol) et d'un agent liant. De tels comprimés à croquer, même s'ils présentent une très forte dureté à l'état solide, sont dissous en milieu aqueux après une courte période de temps dans la bouche et libèrent ainsi rapidement l'actif dans l'organisme.

La fabrication de comprimés matriciels destinés à libérer de façon retardée une substance active dans l'organisme et présentant par ailleurs une forte dureté est enseignée par le brevet US 6,592,901. Dans ce document, des comprimés qui présentent de bonnes caractéristiques de compressibilité, sont obtenus à base d'un grade particulier d'éthylcellulose (éthyl Ether de cellulose non ionique et pH indépendant vendu sous la marque Aqualon®) fortement substituée et de faible viscosité. La résistance à l'écrasement des comprimés ainsi obtenus est de l'ordre de 10 à 20 kp (kiloponds), ce qui, ramené à la taille des comprimés équivaut à environ 1,4-2,8 MPa. Par ailleurs, ce grade spécial d'éthylcellulose est insoluble dans l'eau, limitant la diffusion des liquides et donc la libération de l'actif dans l'organisme. La libération de l'actif s'opère en effet lentement puisque des comprimés obtenus à partir de ce modèle présentent un profil de libération dans lequel moins de 80% de l'actif et libéré au bout de 24 heures.

Des comprimés matriciels présentant une très forte résistance à l'écrasement ont par ailleurs été décrits dans les travaux de Pontier et *al.* (Pontier et al. Journal of European Ceramic Society, 22 (2002)). Les auteurs montrent notamment qu'il est possible d'obtenir des comprimés matriciels très durs à partir d'excipients minéraux de la famille des phosphates de calcium tels que le phosphate tricalcique ou l'hydroxyapatite par compression directe. Ainsi, à partir d'une poudre de phosphate tricalcique préalablement granulée puis comprimée à des forces de compression de l'ordre de 300 MPa, il est possible d'obtenir des comprimés dont la résistance à l'écrasement *(tensile strength)* peut atteindre 6,5 MPa. Cependant cet article ne donne aucune information sur la capacité de tels comprimés à libérer de façon prolongée dans le temps un ou plusieurs principes actifs, ni à la capacité pour de telles structures galéniques à rester intactes en milieu aqueux.

Les travaux de thèse de C. Pontier ("Les phosphates de calcium apatitiques en compression. De la chimie aux qualités d'usage "Thèse de l'Université de Paris XI, soutenue le 25 septembre 2001) montrent qu'il est possible d'obtenir après compression des comprimés matriciels à base de phosphates de calcium (hydroxyapatite et phosphate de calcium tribasique notamment) présentant une très bonne résistance à l'écrasement pouvant aller jusqu'à 7 MPa.

De tels comprimés ont en outre la capacité à libérer la théophylline en milieu aqueux sur une longue période de temps (60% d'actif libéré en 8 heures) par diffusion progressive au travers des pores de la matrice. Cependant cet article ne permet pas de conclure quant aux capacités de tels comprimés à rester intacts en milieu aqueux et donc de résister au détournement par broyage en milieu liquide.

La demande de brevet US 2005/0031546 concerne une forme galénique évitant le détournement toxicomaniaque et comprenant un ou plusieurs principes actifs susceptibles(s) de conduire au développement d'une toxicomanie et au moins un polymère synthétique ou naturel possédant nécessairement une force de rupture d'au moins 500 N. Le seul polymère spécifiquement décrit est le polyoxyde d'éthylène d'un poids moléculaire de 7 000 000 éventuellement associé à une gomme xanthane. Ces comprimés peuvent être préparés par un procédé comprenant une étape de compression précédée d'une étape d'exposition à la chaleur, concomitante avec une étape d'exposition à la chaleur ou suivie d'une étape d'exposition à la chaleur. Ainsi l'étape d'exposition à la chaleur est nécessaire à l'obtention de la dureté désirée. Cette étape, même brève, d'une part n'est pas applicable aux actifs sensibles à la chaleur et d'autre part, nécessite l'utilisation d'un appareillage particulier et un surcoût énergétique qui contribuent à alourdir le coût du procédé.

Il existe donc un besoin réel pour la mise au point d'une forme pharmaceutique qui permette l'administration sécurisée de principes actifs à effet psychotrope de façon prolongée dans le temps, c'est à dire qui présente une structure galénique qui rende mal aisé voire impossible tant son broyage que sa dissolution mais encore qui empêche l'extraction et la séparation du principe actif des agents responsables de la libération prolongée. Cette forme pharmaceutique doit par ailleurs être fabriquée au moyen d'un procédé de fabrication extrêmement simple, rapide et peu coûteux.

La demanderesse a découvert de façon surprenante, une nouvelle formulation galénique orale solide préparée de façon simple sous la forme de comprimés matriciels à libération prolongée, à la fois insolubles et ultra-durs. De tels comprimés permettent d'éviter les phénomènes de mésusage involontaire et de réduire, voire d'éliminer le phénomène de détournement ou de mésusage volontaire.

Aussi l'invention a-t-elle pour objet des comprimés matriciels insolubles dans l'eau capables de libérer un ou plusieurs principes actifs dans l'organisme de façon prolongée dans le temps, préférentiellement pendant des périodes supérieures à 12 heures et plus préférentiellement supérieures à 20 heures, comprenant au moins un principe actif susceptible d'être détourné à des fins de toxicomanie dispersé au sein d'une matrice de compression, ladite matrice étant constituée d'au moins un excipient choisi dans le groupe comprenant les polymères retard pH indépendants et insolubles dans l'eau, les excipients minéraux et leurs mélanges, la quantité dudit excipient et les conditions de compression étant choisies de manière à ce que lesdits comprimés présentent une résistance à l'écrasement d'au moins 4 MPa, avantageusement d'au moins 6 MPa.

De manière avantageuse, les conditions de compression n'impliquent pas nécessairement une étape de chauffage du mélange pour compression ou des outils de compression avant ou pendant l'étape de compression proprement dite.

D'une manière préférentielle, les comprimés conformes à l'invention sont utilisés pour la réalisation de formes pharmaceutiques capables de libérer le ou les principes actifs qu'elles contiennent, notamment des d'agents opioïdes, sur une période de 24 heures, rendant possible une administration de ces actifs en une seule prise par jour (once-a-day).

Dans le cadre de la présente invention les termes mésusage volontaire, détournement ou *drug-abuse* sont employés pour désigner toute altération intentionnelle des formes galéniques. En particulier, la notion de mésusage volontaire concerne la réduction sous forme de poudre de comprimés puis l'inhalation de ces poudres ou leur dissolution dans une faible quantité de liquide en vue d'une injection parentérale.

On parle de comprimé matriciel pour désigner un comprimé dont la structure interne est homogène et identique du centre vers la périphérie du comprimé. Ainsi, les comprimés de la présente invention, sont composés d'un mélange homogène de principe actif sous forme de poudre ou de granules et d'une matrice de compression à base d'au moins un excipient choisi dans le groupe comprenant les polymères retard pH indépendants et insolubles dans l'eau, les excipients minéraux et leurs mélanges.

Dans le cadre de la présente invention, on parlera de matrice de compression pour désigner l'ensemble des excipients qui participent à la cohésion du comprimé. Ladite matrice de compression est à la fois insoluble dans l'eau, et possède une certaine perméabilité (matrice hydrophile) ou un réseau poreux (matrice inerte) responsable de la libération progressive de l'actif, qui ne varie pas en fonction des conditions de pH du milieu.

Le terme « mélange pour compression » est employé dans la présente demande pour désigner l'ensemble des constituants du comprimé (le ou les principes actifs, granulés ou non et les constituants de la matrice de compression) avant leur compression sous forme de comprimé.

Dans la présente demande, on emploiera à la fois la notion de résistance à l'écrasement et la notion de dureté pour caractériser les comprimés. La dureté caractérise la force de rupture du comprimé lors d'un test de rupture diamétrale. Un comprimé rond est placé entre deux mâchoires, l'une est fixe, l'autre mobile. La dureté correspond à la force appliquée par la mâchoire mobile et qui provoque la rupture du comprimé en deux parties à peu près égales. Elle s'exprime en Newton (N) ou kilo Newton (kN) (voir Pharmacopée Européenne : ref : 01/2005:20908).

La résistance à l'écrasement est déduite de la mesure de dureté: c'est un paramètre qui tient compte de la surface du comprimé exposée à la force, et correspond à une force de résistance ramenée à l'unité de surface qui sera exprimée en Pascal (Pa) ou Mega Pascal (MPa), 1 MPa correspondant à 1 Newton par $mm^2$. La résistance à l'écrasement est un paramètre particulièrement intéressant pour comparer le comportement de comprimés de surfaces différentes puisqu'il permet de s'affranchir du paramètre dimension du comprimé. Sa formule de calcul est la suivante (d'après « Détermination of tablet strength by the diametral-compression test ». Fell, J.T. ; Newton, J.M. J. Pharm. Sci., 59 (5) : 688-691 (1970)) :

$$Rd = \frac{2 \times F}{\pi \times D \times h}$$

où

Rd est la rupture diamétrale du comprimé (en MPa)
F est la dureté du comprimé (en N)
D est le diamètre du comprimé (en mm)
H est l'épaisseur du comprimé (en mm).

Dans la présente demande, on parlera de polymères "retards" pour désigner les polymères utilisés de façon courante dans l'industrie pharmaceutique pour retarder la libération d'un principe actif dans son milieu de dissolution. Dans la présente demande, les polymères retards employés sont insolubles dans l'eau, ce qui signifie que la libération de l'actif dans le milieu environnant se fait exclusivement selon un phénomène de diffusion simple, sans qu'il y ait érosion ou dégradation progressive du polymère. En effet, ces polymères présentent vis à vis du milieu environnant une certaine perméabilité, responsable de la diffusion progressive de l'actif hors de la matrice polymérique. Ainsi, plus la perméabilité du polymère est faible, plus la diffusion de l'actif est retardée.

Dans le cadre de la présente invention, on parlera de polymères pH indépendants pour désigner des polymères capables de former un réseau ou une matrice perméable et dont la perméabilité n'est pas influencée par le pH du milieu environnant.

Les comprimés de l'invention sont des comprimés de très haute dureté (ci après dénommés "comprimés ultra-durs"). Ils présentent une structure qui rend leur broyage inenvisageable par des techniques domestiques classiques et leur dissolution dans un milieu aqueux, même acidifié pratiquement impossible.

Cette extrême dureté s'accompagne également d'une friabilité nulle ou quasi nulle ce qui en fait un support galénique de choix pour des principes actifs sujets au détournement comme les agents psychotropes par exemple. Cette friabilité nulle ou quasiment nulle rend les comprimés pratiquement incassables par des techniques classiques ou domestiques (cuillère, mortier, briquet...)

Les comprimés selon l'invention sont également pratiquement insolubles en milieu aqueux, même à faible pH (pH <3). Ces caractéristiques les rendent difficilement administrables par voie parentérale.

Les comprimés selon l'invention sont en outre insolubles en milieu alcoolique, ce qui permet leur administration même en cas d'ingestion d'alcool et évite le mésusage accidentel.

Les comprimés conformes à l'invention permettent en outre, malgré leur structure externe extrêmement dure et résistante, une libération prolongée dans le temps du ou des principes actifs contenus dans ladite matrice. Les comprimés conformes à l'invention permettent ainsi une libération du principe actif dans l'organisme dont la durée est supérieure à 8 heures, préférentiellement supérieure à 12 heures, encore plus préférentiellement supérieure à 20 heures.

Avantageusement, les comprimés conformes à l'invention sont employés pour la réalisation de formes pharmaceutiques d'agents opioïdes pouvant être administrées une seule fois par jour (formes once-a-day).

Enfin, la structure matricielle du comprimé selon l'invention, constituée d'un mélange d'excipients à effet retard connus et approuvés pour un usage par voie orale et de granules contenant le principe actif est extrêmement simple, ce qui rend sa fabrication industrielle aisée, puisqu'une simple étape de compression du mélange permet sa réalisation, sans qu'il soit nécessaire de chauffer les outils de compression et/ou le mélange pour compression avant ou pendant l'étape de compression proprement dite.

D'une manière avantageuse, la matrice de compression des comprimés conformes à l'invention représentera 50 à 98% en poids du poids total des comprimés, encore plus avantageusement, 85 et 95% en poids du poids total desdits comprimés.

Les excipients utilisés seuls ou en mélange dans la constitution de la matrice des comprimés selon l'invention sont de nature organique ; il s'agit du mélange de cellulose microcristalline et d'acétate de polyvinyle/polyvinylpyrrolidone (80 : 20) (vendu sous le nom de marque Kollidon SR[R] )

Le présent fascicule décrit des comprimés dans lesquels excipients de la matrice de compression peuvent également être de nature minérale ; ils appartiennent alors au groupe comprenant les phosphates de calcium (en particulier les phosphates dicalcique ou tricalcique), les silicates d'aluminium et de silicium, et les carbonates de magnésium.

La matrice de compression des comprimés selon l'invention peut avantageusement être constituée d'un mélange de plusieurs des excipients mentionnés plus haut. Il peut s'agir du mélange de polymères organiques comme la cellulose microcristalline et de dérivés vinyliques en proportions variables. Le présent fascicule décrit également des comprimés dans lesquels la matrice de compression est constituée d'un mélange polymère organique + dérivé minéral comme par exemple un mélange silicate de calcium et de silicium + cellulose microcristalline en proportions variables.

Les excipients présents dans la matrice de compression des comprimés conformes à la présente invention représentent avantageusement entre 40 et 100% en poids du poids total de ladite matrice, avantageusement 50 à 90% en poids du poids total de la matrice.

Selon un mode de réalisation avantageux de l'invention, la matrice de compression est constituée d'un mélange (1:1) de deux polymères, avantageusement elle est constituée d'un mélange (1:1) de cellulose microcristalline et du mélange [acétate de polyvinyle/polyvinylpyrrolidone en proportion 80:20 (vendus sous le nom de marque Kollidon SR®) ] Avantageusement ces deux polymères représentent chacun une proportion en poids de l'ordre de 40% du poids total de ladite matrice de compression.

La matrice de compression peut avantageusement, outre les excipients de la matrice de compression contenir un ou plusieurs excipients destinés soit à favoriser le déroulement du processus de compression tels que des agents anti-collage comme la silice colloïdale, le talc, le stéarate de magnésium, le Polyéthylène Glycol (PEG) ou le stéarate de calcium, soit à améliorer la cohésion des comprimés lors de la compression, tels que les agents liants utilisés classiquement dans cette fonction, en particulier les amidons, les dérivés cellulosiques, soit des agents de charge, soit des lubrifiants, soit des plastifiants, soit des agents de remplissage, soit des édulcorants soit des colorants.

Quand ils sont présents, ces excipients sont utilisés de façon classique à raison de 0,1 à 10 % en poids du poids total de la matrice de compression, préférentiellement de 0,5 à 5 % en poids.

Ladite matrice de compression peut comprendre en outre au moins une des substances (a) à (f) suivante ou leur mélange :

- (a) une substance qui irrite les voies nasale et/ou pharyngée,
- (b) un agent augmentant la viscosité et qui permet la formation d'un gel lorsque le comprimé est dissous dans un minimum d'eau,
- (c) un antagoniste du ou des principe(s) actif(s) susceptible d'être détourné à des fins de toxicomanie,
- (d) une substance émétique,
- (e) un colorant comme agent aversif
- (f) une substance au goût amer.

Lorsque le principe actif est un dérivé opiacé naturel ou de synthèse, l'antagoniste sera avantageusement choisi dans le groupe comprenant la naloxone, la naltrexone, le nalmefene, le nalid, la nalmexone, la nalorphine et la naluphine, ces différents composés étant chacun soit sous une forme pharmaceutiquement acceptable, en particulier sous forme de base, de sel ou sous forme solvatée. Ces antagonistes sont présents à des doses classiquement utilisées, notamment à raison de 0,5 à 100 mg par comprimés.

Dans un mode avantageux de réalisation de l'invention, ledit agent antagoniste est la naloxone ou l'un de ses sels pharmaceutiquement acceptable.

Les comprimés conformes à l'invention sont donc particulièrement intéressants comme réservoirs de principes actifs sujets au détournement et devant faire l'objet d'une libération dans l'organisme d'une durée supérieure à 8 heures, préférentiellement supérieure à 12 heures plus préférentiellement encore, supérieure à 20 heures.

Les principes actifs contenus dans les comprimés selon l'invention peuvent être présents sous toute forme connue de l'homme du métier, notamment sous forme de poudre, de cristaux ou de granules.

Les comprimés conformes à l'invention peuvent contenir un ou plusieurs principes actifs qui peuvent être de toute nature. On choisira avantageusement des principes actifs destinés à être libérés dans l'organisme de façon prolongée, c'est à dire sur des périodes de temps d'au moins 8 heures et préférentiellement supérieure à 12 heures, plus préférentiellement encore, supérieure à 20 heures.

D'une manière préférentielle, les comprimés conformes à l'invention sont utilisés pour la réalisation de formes pharmaceutiques administrables en une seule prise par jour (formes dites once-$\alpha$-day).

En particulier, les comprimés selon l'invention sont tout à fait adaptés à la délivrance prolongée d'actifs susceptibles d'être sujets au détournement à des fins toxicomanes et/ou plus généralement à tous les actifs dont la libération rapide ou instantanée dans l'organisme causée par une altération de la forme galénique pourrait être dangereuse ou fatale pour le consommateur.

Ainsi, les comprimés conformes à l'invention sont préférentiellement employés pour la délivrance prolongée d'actifs appartenant à la famille des agents psychotropes, c'est à dire capables d'agir sur le psychisme par des effets stimulants, tranquillisants ou hallucinogènes.

Ainsi, les principes actifs utilisables dans le cadre de l'invention sont préférentiellement des dérivés et/ou des alcaloïdes de l'opium, naturels ou de synthèse telles que la codéine, la narcéine, la noscapine et leurs sels.

Les actifs utilisables selon l'invention appartiennent en outre au groupe comprenant la morphine, ses dérivés et leurs sels et en particulier les morphinènes tels que la pholcodine, la nalorphine, la codéine, la dihydrocodéine, l'hydromorphone, et les morphinanes tels que la buprenorphine, le butorphanol, le dextromethorphane, la nalbufine, la naltrexone, le naloxone, le nalmefene, l'hydrocodone, l'oxymorphone et l'oxycodone et d'une manière générale tous les analogues de la morphine et tous les analgésiques morphiniques, tels que le fentanyle, le tramadol, l'apomorphine et l'étorphine.

La présente invention s'adresse également aux dérivés d'alcaloïdes, naturels ou de synthèse ayant un effet psychotrope, tels que la cocaïne ou l'héroïne.

Enfin, la présente invention s'adresse également à toutes les substances utilisées actuellement en thérapeutique contre l'addiction aux drogues opiacées et/ou comme traitement de substitution et l'aide au sevrage, telle que la méthadone et la buprénorphine par exemple, très sujettes au détournement.

D'une manière générale, la présente invention est également envisageable pour toutes les autres classes thérapeutiques de médicaments actuellement sujettes au détournement, et notamment les neuroleptiques, les tranquillisants, les hypnotiques, les analgésiques, les anxiolytiques, en particulier la classe des benzodiazépines.

Le ou les principe(s) actif(s) constituant les comprimés selon l'invention peu(ven)t représenter entre 5 et 70% en poids du poids total du comprimé. Avantageusement le ou les actifs représente(nt) 10 à 50% en poids du poids total du comprimé. Le(s) principe (s) actif (s) peu(ven)t être directement introduit(s) dans le mélange pour compression, monté(s) sur supports (obtention de microgranules) ou granulé(s) par voie humide ou sèche (obtention de granules).

Lorsque le ou les principe(s) actif(s) sont présents sous la forme de microgranules, ces microgranules peuvent être obtenus de façon classique par dépôt (montage) du ou des actifs à la surface de supports pharmaceutiquement neutres, tels que des microbilles préfabriquées à base de cellulose ou d'un mélange de sucre et d'amidon et vendues sous le terme "*neutral core*" ou *"sugar spheres"* ou encore des granulés d'autres excipients, comme le lactose par exemple.

Le procédé de dépôt (montage) de l'actif est réalisé de façon classique et connue de l'homme du métier et peut varier en fonction de la nature, de la quantité et de la fragilité du ou des principes actifs à déposer. Ainsi, le dépôt (montage) peut s'effectuer par pulvérisation d'une solution ou suspension du ou des principes actifs à la surface du support neutre ou la pulvérisation du ou des actifs en poudre à la surface du support préalablement humidifié à l'aide d'une solution d'agent liant.

Les granules de principe(s) actif(s) peuvent également être obtenus par granulation par voie sèche ou par voie humide du ou des principes actifs d'intérêt, généralement en présence d'au moins un agent liant et d'un liquide de mouillage le cas échéant, selon des techniques, là encore, bien connues de l'homme de l'art.

Les granules ainsi obtenus sont mélangés avec les excipients de la matrice de compression puis le mélange est comprimé.

La dureté exceptionnelle des comprimés selon l'invention peut être obtenue sans qu'il soit nécessaire de faire subir au mélange pour compression (matrice de compression et principe actif) et/ou aux outils de compression (presse) une étape de chauffage préalablement ou pendant la compression.

Avantageusement, les granules comportant le ou les principes actifs d'intérêt ont un diamètre permettant l'obtention d'un bon rendement de compression c'est à dire généralement compris entre 100 et 600 $\mu$m.

Selon un autre mode de réalisation de l'invention, et lorsque sa granulométrie le permet, le principe actif est mélangé directement avec les excipients constituant la matrice de compression puis le mélange est directement comprimé.

Enfin, un autre mode possible de réalisation de l'invention consiste à mélanger le principe actif avec le ou les excipients de la matrice de compression, puis à granuler ce mélange par voie sèche ou humide afin d'obtenir des granules directement compressibles.

Les comprimés conformes à l'invention peuvent être de toute forme et de taille permettant d'obtenir des comprimés de dureté élevée. Avantageusement, la surface totale du comprimé est inférieure à 150 $mm^2$.

La présente invention est donc adaptée à la fabrication tant de comprimés faiblement dosés en actif que de comprimés fortement dosés.

Selon un mode particulier de l'invention, les comprimés peuvent être pelliculés à l'aide d'un enrobage externe que l'homme de l'art saura adapter en fonction des besoins et de la fonction attribuée à cet enrobage.

Ainsi, l'enrobage externe peut être appliqué à des fins de protection du principe actif, lorsqu'il s'agit d'un actif labile sensible aux pH faibles du milieu gastrique par exemple, on parlera alors d'enrobage gastrorésistant.

Par ailleurs, l'enrobage externe peut être appliqué pour ralentir encore la diffusion du principe actif au travers de la matrice. On pourra utiliser à cette fin, différents grades d'éthylcellulose ou de polymères méthacryliques bien connus de l'homme de l'art.

Enfin, l'enrobage externe peut être utilisé pour modifier l'aspect esthétique (texture, couleur) et/ou la palatabilité (sensation en bouche) du comprimé pour le patient. En particulier, on pourra avantageusement utiliser des excipients tels que des dérivés cellulosiques ou des dérivés acryliques bien connus de l'homme de l'art pour masquer le goût du principe actif si nécessaire.

Un tel enrobage peut donc être constitué d'un mélange d'un ou plusieurs excipients connus de l'homme de l'art de nature différente, utilisés seuls ou en mélange pour les différentes fonctions énumérées plus haut.

Le ou les excipients utilisés pour l'enrobage sont appliqués de façon connue de l'homme du métier en quantité nécessaire pour obtenir la ou les fonctions recherchées.

Ces excipients peuvent être appliqués à la surface du comprimé de façon classique par pulvérisation d'une solution ou suspension de l'agent d'enrobage dans un solvant, en turbine perforée ou en lit fluidisé par exemple.

La présente invention concerne en outre le procédé de fabrication des comprimés conformes à l'invention. Ce procédé comporte les étapes suivantes :

- mélange du ou des principes actifs avec le ou les excipients de la matrice de compression,
- éventuellement granulation et
- compression dudit mélange dans des conditions choisies de manière à ce que ledit comprimé présente une résistance à l'écrasement d'au moins 4 MPa, avantageusement d'au moins 6 MPa,
- éventuellement enrobage du comprimé.

Lorsque le polymère d'enrobage du comprimé est un polymère retard, les comprimés enrobés conformes à l'invention peuvent avantageusement subir une phase de maturation dudit polymère d'enrobage afin de garantir sa stabilité physique et chimique. Cette étape est réalisée dans des conditions de température maîtrisée, inférieure à la température de fusion du principe actif pendant un temps contrôlé qui dépend du polymère d'enrobage et qui peut être compris entre 1 minute et plusieurs mois, pour un taux d'humidité relatif de 50 à 99%. Cette étape peut être réalisée en étuve ou en turbine.

L'actif peut-être mélangé directement dans la matrice de compression ou être mélangé sous forme de granules ou de microgranules préparés au préalable. Cette étape de granulation améliore l'uniformité de teneur des comprimés fabriqués. Elle est réalisée de façon préférentielle en voie humide (aqueuse ou organique) pour les granules ou par dépôt (montage) du principe actif en solution ou en suspension sur supports neutres pour les microgranules.

La compression est réalisée sur machine à comprimer rotative avec station de pré-compression. Les paramètres de compression doivent être choisis pour permettre de générer des comprimés de dureté adaptée à la présente invention. Toutefois, il n'est pas nécessaire de faire subir au mélange pour compression ou aux outils de compression une quelconque étape de chauffage avant et/ou pendant la compression dans le but d'atteindre l'exceptionnelle dureté observée sur les comprimés conformes à l'invention. Les forces de compression appliquées sont comprises entre 10 kN et 160 kN, avantageusement entre 30 kN et 80 kN. Elles sont choisies pour être compatibles avec le matériau des poinçons et pour être utilisables à cadences industrielles, tout en permettant d'obtenir des comprimés dont la résistance à la rupture est supérieure à 4 MPa, et préférentiellement supérieure à 6 MPa.

Les exemples 1 à 10 et les figures 1 à 14 qui suivent ont pour but d'illustrer l'invention et ne sauraient en limiter la portée.

La figure 1 représente le profil de dissolution en milieu tampon phosphate pH 6,8 (phosphate monopotassique / phosphate disodique) de comprimés d'oxycodone HCl à 40 mg non pelliculés, obtenus selon l'exemple 1.

La figure 2 représente le profil de dissolution à pH 6,8 de comprimés d'oxycodone HCl à 40 mg non pelliculés, obtenus selon l'exemple 2.

La figure 3 représente le profil de dissolution en milieu tampon phosphate pH 6,8 (phosphate monopotassique / phosphate disodique) de comprimés conformes à l'exemple 2, pelliculés avec une couche d'éthylcellulose EC30 D et ayant subi une étape de maturation dans les conditions de l'exemple 3.

La figure 4 représente les profils de dissolution comparatifs de comprimés matriciels d'oxycodone conformes à l'invention dans un milieu HCl 0,1 N dépourvu d'éthanol et dans un milieu HCl 0,1 N comprenant 40 % d'éthanol tels que mesurés selon l'exemple 4.

La figure 5 illustre les profils de dissolution de comprimés matriciels d'oxycodone conformes à l'invention dans deux milieux de dissolution de pH différents (1,2 et 6,8) selon le mode opératoire décrit dans l'exemple 4.

La figure 6 illustre les profils de dissolution sur 24 heures de comprimés d'oxycodone dosés à 40 mg conformes à l'invention après une période de stockage en blisters alu/alu en conditions de stabilité accélérée de 1 mois, 2 mois, 3 mois et 6 mois dans les conditions de l'exemple 4.

La figure 7 illustre les profils de dissolution sur 24 heures de comprimés d'oxycodone dosés à 20 mg conformes à l'invention après une période de stockage en bouteilles HDPE avec un déshydratant en conditions de stabilité accélérée de 1 mois, 2 mois et 3 mois.

La figure 8 représente les profils plasmatiques d'oxycodone après administration unique de comprimés d'oxycodone dosés à 40 mg conformes à l'invention et de comprimés d'oxycodone du produit de référence Oxycontin®, dosés à 40 mg selon l'exemple 4.

La figure 9 illustre le profil de dissolution sur 24 heures de comprimés non enrobés ultra-durs d'oxycodone et de naloxone à pH 6,8 préparés selon l'exemple 5.

La figure 10 illustre les profils de dissolution sur 10 heures de comprimés non enrobés ultra-durs d'oxycodone dosés à 20 mg à pH 6,8.

La figure 11 illustre les profils de dissolution observés de comprimés conformes à l'invention (« QD ») et de comprimés du produit de référence Oxycontin® (réf) à pH 6,8, pour des comprimés entiers, coupés en deux ou broyés (« en morceaux »)

La figure 12 représente le profil de dissolution de microgranules d'hydromorphone en milieu aqueux (HCl 0,1 N) dans trois conditions de dissolution : sans éthanol, en présence de 20% d'éthanol et en présence de 40% d'éthanol pendant 4 heures selon les conditions de l'exemple 9.

La figure 13 présente le profil de dissolution de microgranules de sulfate de morphine dans un milieu de dissolution à pH 6,8 sans éthanol, à pH 6,8 en présence de 20% d'éthanol et à pH 6,8 en présence de 40% d'éthanol pendant 12 heures selon l'exemple 10.

La figure 14 illustre les tests de dissolution comparatifs entre des comprimés ultra-durs de sulfate de morphine conformes à l'invention et des comprimés de sulfate de morphine du commerce (Avinza®) en présence de A) 20% d'éthanol dans un milieu HCl 0,1 N ou B) 40% éthanol dans un milieu HCl 0,1 N selon l'exemple 10.

**Exemple 1: Fabrication de comprimés à base de granules obtenus par granulation d'oxycodone HCl et d'HPMC à 4,87% et d'une matrice de compression constituée d'un mélange (1 :1) de deux excipients [cellulose microcristalline et (PVA/povidone 80:20)]**

**1. Préparation des comprimés**

***1.1. Préparation des granules d'oxycodone***

Les granules sont obtenus par granulation humide du principe actif (oxycodone HCl, lot N° DV000165 ; Mc Farlan Smith, Angleterre) et de hydroxypropylméthylcellulose (HPMC grade Pharmacoat® 606, Brenntag) qui joue le rôle de liant. La granulation est réalisée en lit fluidisé (GCPG-1, Würster, Glatt, Allemagne) par projection en mode bottom-spray d'une solution du liant (HPMC) sur le principe actif sous forme de poudre.

L'oxycodone est introduite dans la cuve du lit fluidisé et mise en sustentation. La solution liante est pulvérisée sur la poudre qui s'agglomère pour former les granules. L'eau est éliminée progressivement par évaporation puis à l'issue d'une étape finale de séchage. L'étape finale de séchage en étuve (16 heures à 60°C) est réalisée pour obtenir une teneur en eau finale acceptable (inférieure à 6%).

Les proportions d'HPMC et d'oxycodone figurent dans le tableau 1.

Tableau 1

| Numéro de lot des granules | XOXY4979 | |
|---|---|---|
| Ingrédients | Pourcentage [%] | Masse en gramme /lot |
| Oxycodone HCl | 95,13 | 500,0 |
| HPMC (Pharmacoat® 606) | 4,87 | 25,6 |

(suite)

| Numéro de lot des granules | XOXY4979 | |
|---|---|---|
| Ingrédients | Pourcentage [%] | Masse en gramme /lot |
| Eau purifiée | -- | 336,9 |
| Total (sec) | 100,0 | 525,6 |

Les paramètres du procédé de granulation figurent dans le tableau 2 ; la phase 1 correspond à la pulvérisation des premiers 175 g de solution, la phase 2 correspond à la pulvérisation des 185 g restants:

Tableau 2

| Numéro de lot de granules | XOXY4979 | |
|---|---|---|
| Etape | 1 | 2 |
| Température en entrée (°C) | 40 | 45 |
| Température en sortie (°C) | 23-29 | 24-27 |
| Température du produit (°C) | 21-28 | 25-27 |
| Pression de pulvérisation (bar) | 1,0 | 1,2 |
| Vitesse de pulvérisation (g/min) | 10,0 | 6, 0 |
| Etape de séchage (étuve) | 16 heures à 60° C | |

Les granules obtenus à l'issue de l'étape en lit fluidisé présentent les caractéristiques mentionnées dans le tableau 3.

Tableau 3

| Numéro du lot | Taille moyenne des particules (μm) | Masse volumique apparente g/mL | Temps d'écoulement (Sec./100g) | Humidite relative (%) |
|---|---|---|---|---|
| XOXY4979 (HPMC à 4,87%) | 108,7 | 0,450 | 6 | 3,47 |

***1.2. Préparation* de *la matrice de compression***

Un pré-mélange de cellulose microcristalline (Avicel® PH102, FMC) et silice précipitée (Syloïd® 244, Keyser & Mc Kay) est réalisé en mélangeur cubique (AR 401, Erweka) pendant 2 min à 40 rpm. Le mélange polyvinylacétate/povidone (80:20) (Kollidon® SR, BASF) et les granules d'oxycodone préparés comme décrit à l'étape 1.1 sont ajoutés au pré-mélange et l'homogénéisation est réalisée en mélangeur cubique pendant 15 minutes à 40 rpm. Enfin le lubrifiant (stéarate de magnésium, Quimdis) destiné à limiter le collage et les frictions en compression est ajouté au mélange précédent selon les paramètres de mélange : 5 minutes à 40 rpm.

La quantité de granules d'oxycodone utilisés est déterminée de manière à fabriquer des comprimés dosés à 40 mg d'oxycodone.

Les proportions de chacun des excipients sont récapitulées dans le tableau 4.

Tableau 4

| Numéro du lot | XCOX5009 | |
|---|---|---|
| Ingrédients | Pourcentage [%] | Masse (mg/comprimé) |
| Granules d'oxycodone (lotXOXY4979) | 19, 83 | 44, 62 |
| Kollidon® SR | 39,74 | 89,40 |
| Avicel® PH102 | 39,73 | 89,40 |

(suite)

| Numéro du lot | XCOX5009 | |
|---|---|---|
| Ingrédients | Pourcentage [%] | Masse (mg/comprimé) |
| Syloïd® 244 | 0,20 | 0,45 |
| Stéarate de magnésium | 0,50 | 1,13 |
| Total | 100,00 | 225,00 |

### *1.3. Compression*

L'étape de compression du mélange final obtenu à l'étape précédente est réalisée sur une presse à comprimer (PR-12), Sviac) sous une force de compression de 35 kN avec des poinçons oblongs de 11 mm x 5 mm. La compression s'effectue de manière conventionnelle sans que ni le mélange pour compression, ni les outils de compression ne soient soumis à une étape de chauffage avant ou pendant l'étape de compression proprement dite.

Les caractéristiques des comprimés obtenus sont récapitulées dans le tableau 5. Les valeurs moyennes correspondent à la moyenne calculée pour 20 comprimés.

Tableau 5

| Numéro de lot des comprimés | XCOX5009 |
|---|---|
| Masse (mg) | 225 |
| Forme | oblongue |
| Dimension (mm) | 11 x 5 |
| Epaisseur (mm) | 4,15 |
| Dureté (N) | 381 |
| Résistance à l'écrasement (MPa) | 6 |
| Friabilité (%) | 0,0 |

Les comprimés obtenus conformément à l'exemple 1 présentent une très forte résistance à l'écrasement égale à 6 MPa et une friabilité nulle, sans qu'il ait été nécessaire de chauffer les constituants de la matrice ou la presse à comprimer avant ou pendant la compression.

### *1.4. Profil de dissolution des comprimés obtenus selon l'exemple 1*

Les comprimés obtenus selon l'exemple 1 présentent des caractéristiques de dureté et de friabilité qui les rendent pratiquement incassables et en font donc d'excellents candidats à un support galénique permettant de limiter leur détournement par broyage.

De surcroît, la demanderesse a mis en évidence que ces comprimés sont pratiquement insolubles en milieu aqueux, même acide : au terme des essais de dissolution (sur 24 h) les comprimés demeurent intacts au fond du vase de dissolution, à la fois en milieu tamponné à pH 6,8 et en milieu acide pH 1,2.

### 2. Méthode de dissolution

La mesure de dissolution des comprimés obtenus selon l'exemple 1 s'effectue dans 900 mL de milieu de dissolution tamponné à pH 6,8, (phosphate monopotassique / phosphate disodique) selon la méthode de la palette tournante avec une vitesse de rotation des pales de 100 rpm ("*type II paddle apparatus*" conforme à la pharmacopée américaine USP 24).

Le milieu de dissolution est analysé en continu par chromatographie (HPLC) par détection en UV. Pour chaque échantillon la mesure est effectuée sur au moins trois cuves.

Les résultats des tests de dissolution sont récapitulés sur la figure 1.

Ainsi de façon surprenante on observe que les comprimés selon l'invention bien qu'ils soient insolubles ont tout de même la capacité de libérer l'actif qu'ils contiennent de façon prolongée, c'est-à-dire pendant des périodes supérieures à 8 heures préférentiellement supérieures à 12 heures plus préférentiellement encore, supérieure à 20 heures.

De tels comprimés sont donc particulièrement intéressants pour la réalisation de formes pharmaceutiques de type « Once-A -Day », c'est-à-dire ne nécessitant qu'une prise par jour.

**Exemple 2: Fabrication de comprimés à base de granules obtenus par granulation d'oxycodone et d'HPMC à 6,46 % et d'une matrice de compression constituée d'un mélange (1 :1) de deux excipients (cellulose microcristalline et PVA/povidone 80:20)**

Dans cet exemple, la demanderesse a cherché à connaître l'influence de la quantité de liant utilisé lors de l'étape de granulation sur le profil de dissolution des comprimés.

L'étape de granulation est identique à celle décrite pour la réalisation des comprimés conformes à l'exemple 1, à ceci près que la quantité de liant (HPMC, Pharmacoat® 606) est cette fois de 6,46 % en poids du poids total des granules. La composition de ces granules est récapitulée dans le tableau 6.

Tableau 6

| Numéro de lot des comprimés | XOXY5103 | |
|---|---|---|
| Ingrédients | Pourcentage [%] | Masse (g/lot) |
| Oxycodone HCl | 93,54 | 590,5 |
| HPMC (Pharmacoat® 606) | 6,46 | 40,8 |
| Eau purifiée | -- | 483,9 |
| Total (sec) | 100,0 | 631,3 |

Les étapes de mélange et de compression sont ensuite réalisées exactement selon les mêmes paramètres que dans l'exemple 1 selon la même formule qualitative et quantitative.

Les caractéristiques des comprimés obtenus selon l'exemple 2 sont récapitulées dans le tableau 7. Les valeurs moyennes correspondent à la moyenne calculée pour 10 ou 20 comprimés.

Tableau 7

| Numéro de lot des comprimés | XCOX5111 |
|---|---|
| Poids (mg) | 227,0 |
| Forme | Oblongue |
| Dimension (mm) | 11 x 5 |
| Epaisseur (mm) | 4,2 |
| Dureté (Newtons) | 397 |
| Résistance à l'écrasement (MPa) | 6 |
| Friabilité (%) | 0,0 |

Les comprimés obtenus conformément à l'exemple 2 présentent une très forte résistance à l'écrasement égale à 6 MPa et une friabilité nulle. Aucune étape de chauffage avant ou pendant la compression n'a été nécessaire à l'obtention de comprimés d'une telle dureté.

Le profil de dissolution de ces comprimés est ensuite déterminé comme décrit dans l'exemple 1. Ce profil est représenté sur la figure 2.

La quantité de liant utilisée a peu d'influence sur la cinétique de libération qui s'étend sur 24 h.

**Exemple 3: Comprimés obtenus selon l'exemple 2 pelliculés à l'aide d'un enrobage externe d'Aquacoat® ECD-30 (Ethylcellulose)**

Dans cet exemple, l'influence d'un enrobage externe appliqué sur des comprimés d'oxycodone obtenus conformément à l'exemple 2 est évaluée. Là encore, aucune étape de du mélange pour compression ou des outils de compression n'a été réalisée avant ou pendant la compression proprement dite.

## 1. Préparation des comprimés

### *1.1. Sous-enrobage*

Préalablement à l'enrobage par le polymère proprement dit, une étape de sous-enrobage est réalisée sur les comprimés de l'exemple 2.

Cette sous-couche est destinée à améliorer l'état de surface des comprimés. Elle est constituée d'un mélange d'HPMC (Pharmacoat® 603), d'un agent anti-mousse (Siméthicone, Dow Corning), d'un lubrifiant (talc micronisé, Luzenac (Univar)) et d'un agent anti-statique (Syloid 244, Keyser & McKay) de telle sorte que l'HPMC représente un gain en poids de 3% par rapport au poids total des comprimés nus. Les proportions de chacun des excipients figurent dans le tableau 8.

Tableau 8

| Numéro de lot des comprimés | XCOX5112.1 | | |
|---|---|---|---|
| Ingrédients | Pourcentage [%] | Masse / turbine (g) | Masse (mg/comprimé) |
| Comprimés XCOX5111 | 95, 96 | 1000,0 | 227,00 |
| HPMC (603) | 2,88 | 30,0 | 6, 81 |
| Siméthicone (poids sec) | 0,01 | 0,1 | 0, 02 |
| Talc | 0,86 | 9,0 | 2,03 |
| Syloïd® 244 | 0,29 | 3, 0 | 0, 69 |
| Eau purifiée** | N/A | 308,5 | N/A |
| Total (sec) | 100,00 | 1042,07 | 234,5 |
| **Note: l'eau est éliminée durant le procédé ;<br>N/A: Non Applicable | | | |

Ce sous-enrobage est réalisé de manière classique en turbine perforée (Trislot).

Les paramètres du procédé de montage sont récapitulés dans le tableau 9.

Tableau 9

| Numéro du lot des comprimés | XOXY5112.1 |
|---|---|
| Température en entrée (°C) | 38 |
| Temp en sortie (°C) | 32 |
| Vitesse de rotation de la turbine (rpm) | 15 |
| Débit d'air ($m^3$/h) | 150 |
| Pression de pulvérisation (MPa) | 0,12 |
| Débit de pulvérisation (g/min) | 2,0-2,6 |

### *1.2. Enrobage*

L'enrobage proprement dit des comprimés préalablement sous-enrobés est également réalisé en turbine perforée (Trislot).

L'enrobage est réalisé à partir d'une dispersion aqueuse d'éthylcellulose (Aquacoat® ECD-30, FMC) avec une proportion d'éthylcellulose représentant 2,87% en poids du poids total des comprimés enrobés. La proportion des différents excipients figure dans le tableau 10. Là encore, aucune étape spécifique de chauffage des comprimés n'est réalisée avant ou pendant l'application du sous-enrobage ou de l'enrobage proprement dit.

Tableau 10

| Numéro de lots des comprimés | XCOX5112.2 | |
|---|---|---|
| Ingrédients | Pourcentage [%] | poids / turbine (g) |
| Comprimés du lot XCOX5112.1 | 95,75 | 1042,09 |
| Aquacoat® ECD-30 (sec) | 2,87 | 31,24 |
| Dibutyl sebacate | 0, 69 | 7,51 |
| Talc | 0,52 | 5, 66 |
| Syloïd® 244 | 0,17 | 1,85 |
| Eau purifiée** | N/A | 185,04 |
| Total (sec) | 100,00 | 1088,35 |
| **Note: l'eau est éliminée pendant le procédé; N/A: Non Applicable | | |

Les paramètres du procédé de montage sont reproduits dans le tableau 11.

Tableau 11

| Numéro de lot des comprimés | XCOX5112.2 |
|---|---|
| Température d'entrée (°C) | 40 |
| Température de sortie (°C) | 34 |
| Vitesse de rotation de la turbine (rpm) | 15 |
| Débit d'air ($m^3$/h) | 140 |
| Pression de pulvérisation (MPa) | 0,12 |
| Débit de pulvérisation (g/min) | 1,5-2,0 |
| Étape de maturation | XCOX4976.2 |
| Température d'entrée (°C) | 75 |
| Température de sortie (°C) | 65 |
| Température du produit (°C) | 60 |
| Vitesse de rotation de la turbine (rpm) | 3 |
| Débit d'air ($m^3$/h) | 140 |
| Temps (heures) | 24 |

***1.3. Etape de maturation***

Elle est réalisée dans la turbine perforée à la suite de l'enrobage, pendant 24 heures à 60°C, pour permettre la stabilisation du film d'enrobage.

Les comprimés subissent une étape de maturation prolongée (3 mois) à 40°C et 75% d'humidité afin que leur dureté augmente de manière à empêcher leur broyage par des techniques classiques (sous un briquet ou une cuillère) mais également par des techniques moins classiques mais plus efficaces (à l'aide d'un mortier, d'une pince ou d'un marteau par exemple).

Les comprimés ainsi durcis ont une dureté supérieure à 500 N, ce qui équivaut à une résistance à l'écrasement supérieure à 7,4 MPa. Dans ces conditions, la libération de l'actif est maintenue avec plus de 90% d'actif libéré à 24 h comme représenté sur la figure 3.

**Exemple 4 : Comprimés enrobés d'oxycodone alcool-résistants et pH indépendants**

On prépare des comprimés enrobés d'oxycodone à libération retardée, dosés à 40 mg (lot technique n° XCOX5111).
Comme dans l'exemple 1, l'oxycodone est d'abord granulée en lit d'air fluidisé (GPCG1) en présence d'eau et d'un agent liant (HPMC 606).

**4.1. Préparation des comprimés**

***4.1.1. Préparation* de *la matrice de compression***

Un pré-mélange de cellulose microcristalline (Avicel® PH102, FMC) et silice précipitée (Syloïd® 244, Keyser & Mc Kay) est réalisé en mélangeur cubique (AR 401, Erweka) pendant 2 min à 40 rpm. Le mélange polyvinylacétate/povidone (80:20) (Kollidon® SR, BASF) et les granules d'oxycodone sont ajoutés au pré-mélange précédent et l'homogénéisation est réalisée en mélangeur cubique pendant 15 minutes à 40 rpm. Enfin le lubrifiant (stéarate de magnésium, Quimdis) destiné à limiter le collage et les frictions en compression est ajouté au mélange précédent selon les paramètres de mélange : 3 minutes à 40 rpm.
La quantité de granules utilisés est déterminée de manière à fabriquer des comprimés dosés à 40 mg d'oxycodone.
Les proportions de chacun des excipients sont récapitulées dans le tableau 12 ci-dessous.

Tableau 12

| Numéro du lot | XCOX5111 | | |
|---|---|---|---|
| Ingrédients | Fonction | Pourcentage [%] | Masse (mg/comprimé) |
| Granules d'Oxycodone HCl | Actif granulé | 20,25 | 45,56 |
| Kollidon® SR | Agent retard | 39,53 | 88, 93 |
| Avicel® PH102 | Agent retard | 39,53 | 88, 93 |
| Syloïd® 244 | Agent d'écoulement | 0,20 | 0,45 |
| Stéarate de magnésium | Lubrifiant | 0,50 | 1,13 |
| Total | | 100,00 | 225,00 |

***4.1.2. Compression***

L'étape de compression du mélange final obtenu à l'étape précédente est réalisée sur une presse à comprimer (PR-12, Sviac) sous une force de compression de 35 kN avec des poinçons oblongs dont les dimensions figurent dans le tableau ci-dessous.
La compression s'effectue de manière conventionnelle sans que ni le mélange pour compression, ni les outils de compression ne soient soumis à une étape de chauffage avant ou pendant l'étape de compression proprement dite.
Les comprimés dosés à 40 mg d'oxycodone issus de cette étape présentent les caractéristiques suivantes qui sont rassemblés dans le tableau 13 :

Tableau 13

| Numéro de lot des comprimés | XCOX5111 |
|---|---|
| Masse (mg) | 225 |
| Dimension (mm) | 11 x 5 |
| Forme | oblongue |
| Epaisseur (mm) | 4,2 |
| Surface ($mm^2$) | 55 |
| Dureté (N) | 350 |
| Résistance à l'écrasement (MPa) | 5,2 |

(suite)

| Numéro de lot des comprimés | XCOX5111 |
|---|---|
| Friabilité (%) | 0,0 |

On constate donc que les comprimés conformes à l'invention présentent une très forte résistance à l'écrasement, supérieure à 5 MPa.

D'autres comprimés dosés à 20, 40 et 80 mg sont réalisés selon un procédé différent : les granules d'oxycodone sont préparés en granulateur à haut cisaillement. Le mélange pour compression est réalisé comme décrit dans les exemples 1 et 2. Les comprimés sont fabriqués sur machine à comprimer rotative SVIAC PR12, avec des poinçons oblongs de dimensions différentes selon les dosages à fabriquer, sous une force de compression de l'ordre de 10 à 15 kN.

Leurs caractéristiques physiques figurent dans le tableau 14 ci-dessous :

Tableau 14

| Dose | Masse du comprimé | Dimensions L x l x e | Dureté (Résistance à l'écrasement) |
|---|---|---|---|
| 20 mg | 175 mg | 11,0 x 5,0 x 3,8 mm | 300 N (4,9 MPa) |
| 40 mg | 225 mg | 11,0 x 5,0 x 4,2 mm | 350 N (5,2 MPa) |
| 80 mg | 325 mg | 13,0 x 6,0 x 4,5 mm | 400 N (5,6 MPa) |

Les comprimés ainsi fabriqués présentent tous une excellente résistance à l'écrasement, qui est supérieure à 6 MPa quelle que soit leur taille, bien qu'à aucun moment du procédé il n'ait été nécessaire de chauffer les constituants des comprimés ou les outils de compression pour en augmenter la dureté et la résistance.

Les comprimés « nus » dosés à 40 mg issus de l'étape de compression sont ensuite enrobés avec un enrobage destiné à ralentir leur profil de libération dans l'organisme.

***4.1.3. Enrobage***

L'enrobage des comprimés est réalisé en turbine perforée (Trislot).

L'enrobage est réalisé à partir d'une dispersion aqueuse d'éthylcellulose (Aquacoat® ECD-30, FMC) avec une proportion d'éthylcellulose représentant 2,87% en poids du poids total des comprimés enrobés.

Une étape de maturation du film d'enrobage est réalisée en étuve à 60°C pendant 24 h.

La proportion des différents excipients et la formule générale des comprimés enrobés ainsi obtenus figurent dans le tableau 15 qui suit.

Tableau 15

| Numéro de lot | XCOX5112 | |
|---|---|---|
| | Pourcentage | mg /comprimé |
| Oxycodone (DV000165) | 17,40 | 42, 98 |
| HPMC 606 | 1,20 | 2, 97 |
| Kollidon SR® | 36,32 | 89,73 |
| Avicel PH102 | 36,32 | 89,73 |
| Stéarate de magnésium | 0,46 | 1,13 |
| HPMC 603 | 2,76 | 6, 81 |
| Simethicone 30% (VS) | 0,01 | 0, 02 |
| Aquacoat ECD-30 (vs) | 2,87 | 7,08 |
| DBS | 0, 69 | 1,70 |
| Talc micronisé | 1,35 | 3,34 |
| Syloid 244FP | 0, 63 | 1,57 |

(suite)

| Numéro de lot | XCOX5112 | |
|---|---|---|
| | Pourcentage | mg /comprimé |
| Total | 100 | 247,06 |

D'autres comprimés nus dosés à 20, 40, 80 et 160 mg sont également enrobés selon le procédé décrit plus haut.

Leurs caractéristiques physiques observées après enrobage figurent dans le tableau 16 ci-dessous :

Tableau 16

| Dose | Masse du comprimé | Dimensions L x l x e | Dureté (Résistance à l'écrasement) |
|---|---|---|---|
| 20 mg | 175 mg | 11 x 5 x 3,8 mm | 440 N (7,3 MPa) |
| 40 mg | 225 mg | 11 x 5 x 4,2 mm | 500 N (7,4 MPa) |
| 80 mg | 325 mg | 13 x 6 x 4,5 mm | 570 N (6,5 MPa) |
| 160 mg | 575 mg | 15 x 7 x 5,8 mm | 800 N (6,3 MPa) |

Les comprimés ainsi fabriqués présentent tous une excellente résistance à l'écrasement, qui est supérieure à 6 MPa quelle que soit leur taille.

**2. Courbes de dissolution en présence ou non d'alcool dans le milieu de dissolution**

Les comprimés à 40 mg enrobés préparés selon l'exemple 4.3 sont testés en dissolution dans deux conditions :

a) En milieu HCl 0,1 N sans éthanol
b) En milieu HCl 0,1 N avec 40% d'éthanol

Les conditions de dissolution sont les suivantes :

Méthode de la palette tournante, vitesse de rotation des pales : 100rpm, volume du milieu : 900 mL, 1 comprimé par vase. L'oxycodone est dosée par spectrophotométrie U.V à 225 nm.

Les résultats des tests de dissolution sont représentés sur la figure 4.

On constate que malgré la présence d'alcool dans le milieu de dissolution, les comprimés conformes à l'invention conservent un profil de dissolution retardé.

**3. Courbes de dissolution en fonction du pH**

Des comprimés dosés à 40 mg fabriqués comme décrit plus haut dans cet exemple ont également été testés sur le plan de leur indépendance vis-à-vis de pH, c'est-à-dire sur leur faculté à conserver un profil de libération constant quelle que soit la valeur du pH du milieu de dissolution.

Deux conditions expérimentales ont été mises en oeuvre :

Milieu de dissolution à pH 6,8
Milieu de dissolution à pH 1,2

Les profils de dissolution obtenus sont représentés sur la figure 5.

On constate que quel que soit l'acidité du milieu de dissolution, les comprimés conformes à l'invention conservent un profil de libération retardé constant.

Ces comprimés peuvent donc être considérés comme pH-indépendants, ce qui leur confère un avantage particulier dans la mesure où ils peuvent être utilisés comme vecteurs pour n'importe quel type de principes actifs devant faire l'objet d'une libération prolongée.

### 4.3. Études de stabilité

#### *4.3.1. Stabilité au stockage*

Les comprimés enrobés dosés à 40 mg d'oxycodone, obtenus selon la méthode décrite plus haut sont ensuite étudiés sur le plan de la stabilité afin de déterminer leur réaction au stockage.

Les comprimés sont maintenus pendant 6 mois en condition de stabilité accélérée selon les normes ICH en vigueur (45°C ; 75% d'humidité) dans deux types de conditionnement : a) blister d'aluminium sur deux faces (alu/alu) et b) bouteilles HDPE (polyéthylène haute densité) en présence d'un agent déshydratant.

Les caractéristiques des comprimés à l'issue de la période de stockage prolongée sont récapitulées dans le tableau 17 qui suit :

Tableau 17

| Conditionnement | Dose initiale | Dosage après stockage mg/comprimé | Impuretés | Dureté | Proportion d'eau |
|---|---|---|---|---|---|
| Blister alu/alu | 40mg | 40,9 CV 0,5% | 0,17% | > 500N | 3,5% |
| Bouteilles HDPE | 20mg | 19,9 CV 3,5% | 0,17% | 440N | 3,6% |

#### *4.3.2. Profils de dissolution obtenus après une période de stockage.*

Ces profils de dissolution sont obtenus dans les conditions suivantes : méthode de la palette tournante, vitesse de rotation des pales : 100 rpm, volume du milieu de dissolution : 900 mL, pH 6,8.

Ils sont donnés dans les figures 6 et 7.

On constate que non seulement la quantité d'actif est conservée au cours du temps, mais que les profils de libération de l'actif et l'extrême dureté des comprimés sont maintenus à l'issue d'une période de stockage de 6 mois.

Les comprimés conformes à l'invention sont donc stables et présentent un profil de dissolution qui est à la fois indépendant du pH et indépendant de la présence (même élevée) d'alcool dans le milieu de dissolution.

### 4.4. Etudes cliniques

Les comprimés dosés à 40 mg fabriqués selon cet exemple sont également testés in vivo afin de déterminer le profil plasmatique de l'oxycodone chez des patients auxquels on a administré lesdits comprimés.

Une étude clinique (Algorithme, Canada, n° OXY/24018/001) est réalisée sur 12 volontaires sains mâles et femelles à jeun séparés en deux demi-groupes. Chaque demi-groupe reçoit successivement les deux traitements (comprimés de l'invention et produit de référence) après une période intermédiaire sans administration (« lavage » ou wash-out).

Le produit de référence utilisé dans cette étude est l'Oxycontin®, comprimé d'oxycodone à libération prolongée administrable à raison de deux prises par jour, également dosé à 40 mg. (lot N°121777, expiration Avril 2007, Purdue).

Les profils plasmatiques de l'oxycodone obtenus sont donnés dans la figure 8 et les paramètres sont rassemblés dans les tableaux 18 et 19 qui suivent :

Tableau 18

| Paramètre | Test (invention) | | Référence | |
|---|---|---|---|---|
| | Moyenne | C.V | Moyenne | C.V |
| $C_{max}$ (ng/mL) | 34,412 | 20 | 53,129 | 25,0 |
| $T_{max}$ (heures) | 10,0 | 16, 6 | 3.00 | 34,3 |
| $AUC_t$ (ng h/ mL) | 667,109 | 16,9 | 611,848 | 21,9 |
| $AUC_\infty$ (ng h/ mL) | 679, 846 | 17,1 | 614,960 | 21,7 |
| $AUC_{t/\infty}$ (%) | 98, 17 | 1,7 | 99,48 | 0,3 |
| $K_{el}$ (heures$^{-1}$) | 0,1154 | 24,0 | 0,1561 | 16,4 |

(suite)

| Paramètre | Test (invention) | | Référence | |
|---|---|---|---|---|
| | Moyenne | C.V | Moyenne | C.V |
| $T_{1/2}$ el (heures) | 6.39 | 28,0 | 4,56 | 17,2 |

Note : Pour les valeurs de $T_{max}$, c'est la valeur moyenne qui est indiquée ;
C.V : Coefficient de variation ;
$K_{el}$ : constante d'élimination ;
$T_{1/2\ el}$ : demi-vie d'élimination

Tableau 19

| Paramètres | Ratio | Intervalle de confiance à 90 % | |
|---|---|---|---|
| | | Inférieur | Supérieur |
| Cmax | 65 | 58 | 73 |
| $AUC_t$ | 110 | 104 | 116 |
| $AUC_\infty$ | 111 | 105 | 118 |

Ainsi, les profils plasmatiques obtenus montrent qu'il n'y a pas de perte de biodisponibilité de l'actif, malgré une diminution du Cmax.

Ainsi, ces comprimés matriciels à base d'oxycodone conformes à l'invention présentent un profil plasmatique après administration unique chez l'être humain, tel que le ratio de leur Cmax sur le Cmax observé après administration de comprimés d'oxycontin® à libération prolongée (« *Oxycontin® extended release)* de même dosage ne dépasse pas 0,7.

Par ailleurs, ces comprimés matriciels à base d'oxycodone selon l'invention, présentent un profil plasmatique après administration unique chez l'être humain, tel que le ratio de l'AUC∞ observée pour ces comprimés sur l'AUC∞ observée pour des comprimés d'oxycontin® à libération prolongée (« *Oxycontin® extended release)* de même dosage est compris dans l'intervalle de bioéquivalence de 80 à 125 %.

Ces résultats sont particulièrement avantageux puisqu'ils signifient que l'oxycodone est aussi bien absorbée par l'organisme que ne l'est le produit de référence, mais que, présentant une concentration maximale diminuée d'environ 35 % pour les comprimés de l'invention, elle permet une réduction substantielle des risques d'effets secondaires qui surviennent à de fortes concentrations plasmatiques.

**Exemple 5 : Comprimés d'oxycodone et de naloxone**

**5.1. Préparation des comprimés**

Des comprimés conformes à l'invention sont fabriqués en associant deux principes actifs : l'oxycodone et la naloxone.

La naloxone est un antagoniste aux opiacées, qui bloque l'activité de l'oxycodone si le comprimé est détourné de son usage pour être pris par injection. En effet, lorsque le comprimé est pris de façon conventionnelle (par voie orale), la naloxone n'exerce pas son effet antagoniste car elle est rapidement métabolisée lorsqu'elle est ingérée par voie orale. Le ratio oxycodone / naloxone base employé ici est de 4:1.

Les comprimés sont réalisés de la même manière que dans l'exemple 4 (granulation de l'oxycodone en granulateur à haut cisaillement). Ils ne subissent aucun traitement thermique avant, pendant ou après la compression.

La formule générale des comprimés ainsi fabriqués (lot XCOX 5731) est récapitulée dans le tableau 20 qui suit.

Tableau 20

| Matières premières | Mg/cp | (%) |
|---|---|---|
| Oxycodone granulée | 22,66 | 12,51 |
| Naloxone $2HCl.H_2O$ | 6,10 | 3,37 |

(suite)

| Matières premières | Mg/cp | (%) |
|---|---|---|
| Kollidon SR® | 75,54 | 41,71 |
| Avicel pH102® | 75,54 | 41,71 |
| Syloïd 244 | 0,367 | 0,20 |
| Stéarate de Magnésium | 0,91 | 0,50 |
| Total | 181,1 | 100,0 |

Les comprimés après compression présentent les caractéristiques physiques rassemblées dans le tableau 21 qui suit.

Tableau 21

| Description comprimé | Rond, plat, blanc |
|---|---|
| Diamètre | 8 mm |
| Epaisseur | 2,90 mm |
| Masse moyenne | 175,8 mg |
| Dureté | 315 N |
| Résistance diamétrale | 8,6 MPa |

On constate qu'il est possible de réaliser, conformément à l'invention, des comprimés de très forte résistance à l'écrasement pouvant contenir deux actifs, en particulier un agent opioïde et un agent antagoniste bloquant son action dans le cas d'une administration du comprimé par voie intraveineuse.

**5.2. Profils de dissolution**

Des tests de dissolution sont réalisés, comme dans les exemples précédents, dans les conditions suivantes : Appareil de type 2 (pales) / 100 rpm / milieu pH 6,8 / volume du milieu de dissolution : 900 mL / dosage par spectrophotométrie UV continu à 225nm / largeur des cuves : 10mm.
Le profil est donné dans la figure 8.
On constate que ces comprimés ultra-durs présentent un profil de dissolution retardé (90% d'actif libéré au bout de 12 heures)

**Exemple 6 : Comprimés à base de dérivés minéraux**

**6.1. Préparation des comprimés**

Le but de cet essai est de réaliser des comprimés dans lesquels on emploie des excipients minéraux comme agent principal de la matrice de compression.
On réalise des comprimés à base d'oxycodone et de dicalcium phosphate dihydrate (Emcompress®) en remplacement des excipients type Kollidon SR® et Avicel PH 102® qui étaient utilisés dans les exemples précédents.
Le procédé de préparation est identique à celui décrit dans l'exemple 1 (granulation de l'oxycodone puis mélange physique avec les excipients du mélange pour compression en poudre).
La formule générale de fabrication de ces comprimés (lot XCOX 5723) dosés à 20mg est présentée dans le tableau 22 qui suit.

Tableau 22

| Matières premières | Mg/cp | (%) |
|---|---|---|
| Oxycodone granulée (XOXY 5634) | 22,57 | 12, 90 |
| Emcompress® | 151,21 | 86,40 |

(suite)

| Matières premières | Mg/cp | (%) |
|---|---|---|
| Syloïd 244FP | 0,35 | 0,20 |
| Stéarate de Magnésium | 0,88 | 0,50 |
| total | 175,0 | 100,0 |

Le mélange ainsi obtenu est comprimé comme dans l'exemple 1.
Les caractéristiques physiques des comprimés après compression sont données dans le tableau 23 qui suit :

Tableau 23

| Description du comprimé | Rond, plat, blanc |
|---|---|
| Diamètre | 6 mm |
| Epaisseur | 3,16 mm |
| Masse moyenne | 178,8 mg |
| Dureté | 170 N |
| Résistance diamétrale | 5,7 MPa |

On constate que là encore, la résistance à l'écrasement obtenue est bien supérieure à 4 MPa, bien qu'aucune étape de chauffage du mélange ou des outils de compression n'ait été nécessaire.

**6.2. Profil de dissolution**

Les comprimés ainsi obtenus sont ensuite placés en milieu de dissolution.
Les conditions de dissolution sont les suivantes : appareil de type 2 (pales) ; vitesse de rotation des pales : 100 rpm ; milieu pH 6,8 ; volume du milieu de dissolution : 900 ml ; UV continu à 225nm ; cuves 10mm.
Les résultats sont donnés dans la figure 9.
On constate que les comprimés obtenus à partir d'excipients minéraux sont capables de libérer l'oxycodone de façon relativement prolongée dans le temps.

**Exemple 8 : Tests de détournement**

**8.1. Essais d'écrasement**

L'objectif de cet exemple est de déterminer la difficulté à casser ou à broyer puis éventuellement à obtenir une poudre à partir de comprimés d'Oxycodone conformes à l'invention par rapport à des comprimés d'oxycodone de référence (Oxycontin®).
Quatre moyens de mise en oeuvre pour cette étape ont été choisis et classés par ordre croissant de difficulté :

- couteau (type Opinel®)
- cuillère à café
- pince universelle (type pince multiple)
- mortier et pilon en verre (verrerie du laboratoire)

L'évaluation de la difficulté d'écrasement a été déterminée en fonction de la dureté du comprimé.
Les caractéristiques physiques des comprimés d'Oxycodone testés sont données dans le tableau 24

Tableau 24

| Comprimé testé | Epaisseur | Taille | Forme | Masse (mg) | Dureté (N) | Résistance à l'écrasement (MPa) |
|---|---|---|---|---|---|---|
| OxyContin® 20mg | 3,43 | Diamètre 7,24 mm | Rond rose | 135,9 | 105 | 2,7 |
| Invention (20 mg) | 3,30 | Longueur 11,0 mm Largeur 5,5 mm | Oblong blanc | 175,9 | 467 | 8,8 |

La résistance à l'écrasement des comprimés de référence est 3,3 fois plus faible que celle du comprimé selon l'invention.

L'utilisation de la pince a permis un broyage grossier des comprimés (morceaux de 1 à 2 mm), aussi bien pour la référence que pour les comprimés conformes à l'invention.

A l'issue de l'étape de broyage grossier à l'aide de la pince, l'utilisation du mortier de laboratoire permet l'obtention d'une poudre fine dans les deux cas. Cependant, l'utilisation du mortier sur les comprimés conformes à l'invention intacts ne permet pas leur écrasement.

Les difficultés d'écrasement observées sur chacun des types de comprimés en fonction de l'outil employé sont rassemblées dans le tableau 25 qui suit :

Tableau 25

| | Couteau | Cuillère à café | Pince | Mortier |
|---|---|---|---|---|
| OxyContin® 20mg | Coupure facile, Effritement | Ecrasement facile | Ecrasement facile, effritement | Ecrasement très facile |
| Invention 20 mg | Coupure difficile, pas d'écrasement | Ecrasement impossible | Ecrasement facile, effritement | Ecrasement impossible (sans coupure préalable) |

La référence OxyContin® s'écrase assez facilement quel que soit le moyen utilisé. Etant donné que sa dureté est faible, il a tendance à s'effriter.

En revanche, le comprimé conforme à l'invention ne s'écrase qu'avec la pince universelle, le couteau permet seulement de le couper mais pas de l'écraser. Après la coupure, les morceaux peuvent être broyés au mortier.

**8.2. Tests de dissolution**

Un comprimé coupé en 2 à l'aide du couteau et un comprimé grossièrement écrasé à l'aide de la pince sont soumis à un test de dissolution pour analyser l'impact de la coupure et du broyage sur le profil de dissolution par rapport au comprimé entier. Ce test est réalisé sur le lot XCOX 5726 préparé selon l'exemple 4 et sur l'Oxycontin® de référence.

La méthode de dissolution est la suivante : dissolution continue, milieu de dissolution à pH 6,8, 900ml de milieu par vase, méthode de la palette tournante, vitesse de rotation des pales : 100 rpm, dosage : 40mg de principe actif par vase, épaisseur des cuves : 10mm, mesure par spectrophotométrie U.V (longueur d'onde $\lambda$ = 225 nm). Les lectures se font toutes les 5 minutes pendant la première heure puis toutes les 15 minutes jusqu'à 24H.

Les résultats des dissolutions dans le milieu à pH 6,8 sont donnés dans le tableau 26 qui suit et dans la figure 11.

Tableau 26

| Temps (h) | Oxycontin® 20mg lot 122810 | | | Oxycodone 20mg XCOX 5726 | | |
|---|---|---|---|---|---|---|
| | Comprimé entier | Comprimé coupé en 2 | Comprimé en morceaux | Comprimé entier | Comprimé coupé en 2 | Comprimé en morceaux |
| 0,5 | 35,9 | 50,8 | 61,0 | 1,3 | 8, 6 | 26,7 |
| 1 | 47,1 | 62,8 | 73,4 | 3,7 | 15,0 | 36,5 |
| 2 | 60,5 | 75,2 | 85,4 | 10,7 | 28,2 | 51,5 |
| 3 | 69,4 | 82,3 | 91, 6 | 17,3 | 39,4 | 62,2 |

(suite)

| Temps (h) | Oxycontin® 20mg lot 122810 | | | Oxycodone 20mg XCOX 5726 | | |
|---|---|---|---|---|---|---|
| | Comprimé entier | Comprimé coupé en 2 | Comprimé en morceaux | Comprimé entier | Comprimé coupé en 2 | Comprimé en morceaux |
| 4 | 76,2 | 87,0 | 95,4 | 24,9 | 49,7 | 70,4 |
| 6 | 86,0 | 92,9 | 99,0 | 41,7 | 64,8 | 81, 9 |
| 8 | 92,8 | 96,5 | 100,3 | 55,8 | 75,3 | 88,8 |
| 12 | 100,7 | 99,4 | 100,7 | 75,7 | 88,1 | 95,9 |
| 16 | 103,4 | 100,1 | 100,5 | 87,7 | 94,7 | 99,2 |
| 20 | 103,9 | 99,4 | 99,5 | 95,3 | 98,4 | 100,7 |
| 24 | - | 98,2 | 99,2 | 100,4 | 100,5 | 101,5 |

On constate que dans le milieu à pH 6,8, le profil de dissolution de la référence est proche de celui de la cible d'un comprimé nu, c'est-à-dire sans enrobage retard alors que le profil du comprimé (« QD ») de l'invention est proche de celui de la cible d'un comprimé à libération retardée.

Le fait de couper en deux le comprimé accélère la dissolution et l'accélération est accentuée lorsque le comprimé est en morceaux pour les deux types de comprimés, rendant le principe actif plus rapidement disponible pour l'absorption par voie orale.

Cependant, le profil de l'Oxycodone du comprimé « QD » broyé, conforme à l'invention, reste un profil retard.

**8.3 Evaluation de l'extraction du principe actif**

Les comprimés testés ont également fait l'objet d'une évaluation sur le plan de l'extraction du principe actif destinée à une injection.

La demanderesse a utilisé le kit dit « Stéribox®>> accessible en pharmacie et destiné à un public toxicomane, dans le but de prévenir la transmission d'agents pathogènes par l'échange de seringue contaminées.

Le Stéribox® contient :

- deux seringues de 1 ml,
- deux dosettes d'eau de 5 ml pour préparations injectables,
- deux coupelles ou « Stéricup® »
- deux filtres

L'extraction de l'Oxycodone du produit de référence et du comprimé conforme à l'invention est réalisée de la manière suivante sur chacun des lots :

- 2 essais sur un comprimé entier,
- 2 essais sur un comprimé grossièrement broyé avec la pince,
- 2 essais sur un comprimé selon l'invention broyé à l'aide de la pince puis du mortier et du pilon et
- 2 essais sur un comprimé sur un comprimé de référence broyé directement au mortier.

Le milieu d'extraction testé est l'eau présente dans le Stéribox®, sous le volume maximum disponible (2 ml).

Le mode opératoire de l'extraction employé est celui qui est décrit dans la notice du Stéribox®.

1- introduire l'échantillon préparé (entier, grossièrement broyé ou broyé) dans la coupelle,
2- introduire 2 ml d'eau à l'aide d'une pipette jaugée,
3- mélanger avec le piston de la seringue pendant 2 minutes,
4- faire chauffer le contenu de la coupelle avec un briquet pendant 1 minute,
5- vérifier le volume restant après chauffage : il reste 1,7ml.
6- filtrer la solution à l'aide du filtre stérile contenu dans le Steribox® qui a été introduit au préalable dans la seringue. S'aider si besoin d'une pipette pour mettre la solution de la coupelle dans la seringue,
7- diluer le filtrat dans l'eau pour avoir une concentration théorique en principe actif de 20mg/100ml
8-effectuer l'analyse en remplaçant le solvant d'extraction par de l'eau pour le témoin et l'essai.

Les résultats des teneurs obtenues et des rendements d'extraction pour chacun des essais sont récapitulés dans le tableau 27 qui suit.

Tableau 27

| | Oxycontin 20mg lot 122810 | | | Oxycodone 20mg XCOX 5726 | | |
|---|---|---|---|---|---|---|
| | Comprimé entier | Comprimé grossièrement broyé | Comprimé broyé | Comprimé entier | Comprimé grossièrement broyé | Comprimé broyé |
| Teneur obtenue mg/cp | 0,37* | 16,3 | 18,4 | 0,25 | 5,8 | 15,2 |
| Cv (%) | - | 4, 6 | 3,3 | 4,1 | 2,1 | 15,9 |
| Rendement | 2,% | 86,2 % | 97,4 % | 1,3 % | 30,4 % | 79,6 % |
| * Le résultat est sur un seul essai, le second étant trouble, le résultat n'est pas exploitable. | | | | | | |

On constate que le rendement d'extraction est faible lorsque le comprimé est encore entier, quel que soit le comprimé employé.

Cependant, les rendements d'extraction sont plus élevés pour l'Oxycontin® dans tous les essais. En particulier, lorsque le comprimé conforme à l'invention est grossièrement broyé, il libère près de 5 fois moins de principe actif que le produit de référence utilisé dans les mêmes conditions.

Ces résultats montrent que le détournement par voie intraveineuse peut être réalisé plus facilement avec l'Oxycontin® qu'avec les comprimés d'Oxycodone « QD » conformes à l'invention.

Une simple pince suffira pour obtenir une bonne extraction du comprimé d'Oxycontin® alors que dans le cas de l'Oxycodone « QD » de l'invention, il faudra un outil supplémentaire pour parvenir à un broyage efficace et augmenter ainsi le rendement d'extraction. Les comprimés conformes à l'invention sont donc particulièrement efficaces pour freiner l'usage détourné à des fins toxicomanes de principes actifs opioïdes.

**Exemple 9 : Microcomprimés d'hydromorphone**

**9.1. Préparation des comprimés**

Dans cet exemple, on réalise des comprimés de forte dureté non enrobés, conformes à l'invention, à base d'hydromorphone, alcaloïde dérivé de la morphine exerçant une puissante action analgésique utilisé dans le traitement de la douleur et donc susceptible d'être détourné à des fins de toxicomanie.

On réalise des microcomprimés de 3 mm de diamètre dosé à 3 mg d'hydromorphone.

***9.1.1. Préparation des ingrédients***

La composition des comprimés est récapitulée dans le tableau 28 ci-dessous.

Tableau 28

| Ingrédients | Pourcentage [%] | Masse (mg/comprimé) |
|---|---|---|
| Hydromorphone HCl | 12 | 3 |
| Kollidon® SR | 50 | 12,5 |
| Avicel® PH102 | 36 | 9 |
| Silice | 1 | 0,25 |
| Stéarate de magnésium | 1 | 0,25 |
| Total | 100,00 | 25,00 mg |

### *9.1.2. Compression*

L'hydromorphone est tout d'abord mélangée aux excipients formant le mélange pour compression (Avicel PH102, Kollidon® SR et silice) après une étape de tamisage destinée à éliminer les particules de grosses taille et autres agglomérats, Le mélange s'effectue à sec dans un mélangeur (V Blender) conventionnel pendant 20 minutes, Puis le stéarate de magnésium en poudre est ajouté au mélange et l'ensemble est à nouveau mélangé pendant 2 à 5 minutes. La compression du mélange final ainsi obtenu est réalisée par compression directe du mélange sur une presse rotative RT6 Sviac à 6 postes, à l'aide poinçons concaves extra-profonds de 3mm de diamètre. La force de compression appliquée est de 17,8 KN.
Les caractéristiques des comprimés ainsi obtenus sont présentées dans le tableau 29 qui suit.

Tableau 29

| Numéro de lot des comprimés | RD1095L1C1 |
|---|---|
| Masse moyenne(mg) | 23,8 mg |
| Épaisseur (mm) | 3,47 |
| Dureté (N) | 108 N |
| Résistance à l'écrasement (MPa) | 6,6 MPa |
| Friabilité (%) | 0,0 |

Les comprimés obtenus ont une dureté supérieure à 108 N, soit une résistance à l'écrasement supérieure à 6,8 MPa et une friabilité nulle.

### *9.1.3 Profil de dissolution*

Le profil de dissolution des comprimés ainsi réalisés est mesuré par spectrophotométrie UV sur 12 heures dans trois conditions :

a) Sans alcool dans le milieu HCl 0,1 N
b) Avec 20% d'alcool dans le milieu HCl 0,1 N
c) Avec 40 % d'alcool dans le milieu HCl 0,1 N

Les conditions de dissolution sont les suivantes :

appareil de type 2 (pales) ; vitesse de rotation des pales : 100 rpm ; volume du milieu de dissolution : 500 ml ; 37°C ; filtre 45 μm, prélèvement automatique (en circuit fermé) aux temps de prélèvement: 5, 15, 30, 45, 60, 120, 180 et 240 minutes. Analyse par UV à 280 nm avec des cellules de 1,0 cm ; 10 comprimés d'hydromorphone sont déposés dans chacun des vases (équivalent de 30 mg/vase).

Les résultats de dissolution sont présentés sur la figure 12.
On constate que dans tous les cas, les comprimés conformes à l'invention présentent un profil retardé qui n'augmente pas du fait de la présence d'éthanol dans le milieu de dissolution,
Les comprimés d'hydromorphone conformes à l'invention présentent donc à la fois une excellente résistance à l'écrasement mais également une bonne résistance à l'alcool. Ils sont donc parfaitement adaptés à des actifs susceptibles de subir un mésusage non intentionnel ou d'être détournés de manière volontaire,

### Exemple 10 : Comprimés de sulfate de morphine

Dans cet exemple, des comprimés ultra durs non enrobés à base de sulfate de morphine ont été fabriqués et leur résistance à l'alcool présent dans le milieu de dissolution a ensuite été comparée à celle d'une formulation de sulfate de morphine vendue dans le commerce sous le nom de marque Avinza®. Les comprimés selon l'invention sont dosés à 30 mg.

### *10.1.1 Formulation*

On prépare tout d'abord des microgranules de sulfate de morphine montés sur des grains neutres d'une taille de 500 à 600 µm à l'aide d'un agent liant : l'HPMC.

Ces microgranules sont ensuite broyés au mortier afin d'obtenir une granulométrie fine permettant un mélange uniforme. Ce broyat est ensuite mélangé avec les excipients de compression afin d'obtenir le mélange permettant de réaliser les comprimés ultra-durs conformes à l'invention. La nature et la proportion de chaque excipient sont récapitulées dans le tableau 30.

Tableau 30

| Numéro du lot | NP062M2L1 | |
|---|---|---|
| Ingrédients | Pourcentage [%] | Masse (mg/comprimé) |
| Microgranules de sulfate de morphine broyés (lot NP062M2) | 19,98 | 72,927 |
| Kollidon® SR | 39,74 | 145,05 |
| Avicel® PH101 | 39,58 | 144,47 |
| Syloïd® 244 | 0,20 | 0,73 |
| Stéarate de magnésium | 0,50 | 1,825 |
| Total | 100,00 | 365,00 |

### *10.1.2. Compression*

La compression du mélange final ainsi obtenu est réalisée sur une presse à comprimer (PR-12, Sviac) avec des poinçons ronds de 10 mm de diamètre à une force de compression de 26,1 kN.

Les caractéristiques des comprimés ainsi obtenus sont présentées dans le tableau 31 qui suit.

Tableau 31

| Numéro de lot des comprimés | NP062M2L1C1 |
|---|---|
| Masse (mg) | 365 |
| Forme | Ronde et plate |
| Diamètre (mm) | 10 |
| Épaisseur (mm) | 3,7 |
| Dureté (N) | > 343,23 |
| Résistance à l'écrasement (MPa) | > 5,9 |
| Friabilité (%) | 0,0 0 |

Les comprimés obtenus ont une dureté supérieure à 343 N soit une résistance à l'écrasement supérieure à 5,9 MPa et une friabilité nulle,

### *10.1.3. Profils de dissolution*

Le profil de dissolution des comprimés ainsi réalisés conformément à l'invention est mesuré par spectroscopie U.V sur 12 heures à pH 6,8 dans trois conditions :

a) Sans alcool dans le milieu,
b) Avec 20% d'alcool dans le milieu
c) Avec 40 % d'alcool dans le milieu

Les conditions de dissolution sont les suivantes : appareil à palette tournantes; volume du milieu de dissolution : 500 mL; vitesse de rotation des pales = 100 rpm; un comprimé de 30 mg par vase.

Les résultats de dissolution sont présentés sur la figure 13.

On constate que dans tous les cas, les comprimés conformes à l'invention présentent un profil retardé qui n'augmente pas du fait de la présence d'éthanol dans le milieu de dissolution. Ces comprimés présentent donc une importante résistance à l'alcool, ce qui procure aux comprimés conformes à l'invention un net avantage sur d'autres comprimés de sulfate de morphine à libération retardée disponibles dans le commerce.
Des tests de dissolution comparatifs ont été réalisés sur des comprimés fabriqués conformément à l'invention et des comprimés du commerce à base de sulfate de morphine (Avinza®). La méthode de dissolution employée est la même que celle décrite précédemment à ceci près que le milieu de dissolution est un milieu HCl 0,1 N. Deux conditions sont testées : milieu HCl 0,1 N en présence de 20 % d'éthanol (A) et milieu HCl 0,1 N en présence de 40% d'éthanol(B).
Les résultats de dissolution sont rapportés sur la figure 14.
Le produit du commerce présente un profil de dissolution très rapide et n'est pas une forme retard. En outre il présente une faible tolérance à l'alcool puisque la vitesse de dissolution augmente avec la quantité d'alcool présente dans le milieu.
En revanche la vitesse de dissolution du comprimé selon l'invention n'est pas modifiée par la présence d'alcool dans le milieu.

**Revendications**

1. Comprimés matriciels insolubles dans l'eau capables de libérer un ou plusieurs principes actifs dans l'organisme de façon prolongée dans le temps et comprenant au moins un principe actif susceptible d'être détourné à des fins de toxicomanie dispersé au sein d'une matrice de compression, ladite matrice étant constituée d'un mélange de cellulose microcristalline et [d'acétate de polyvinyle/polyvinylpyrrolidone (80:20)], **caractérisés en ce que** :

   - ledit principe actif représente 10 à 50 % en poids du poids total du comprimé,
   - ladite matrice de compression représente 50 à 98% en poids du poids total dudit comprimé, avantageusement 85 à 95 %,
   - ledit mélange de cellulose microcristalline et [d'acétate de polyvinyle/polyvinylpyrrolidone (80:20)] représente 40 à 100 %, avantageusement 50 à 90 % en poids du poids total de la matrice, et obtenus en appliquant des forces de compression comprises entre 10 kN et 160 kN.

2. Comprimés matriciels selon la revendication 1 **caractérisés en ce que** ni le mélange pour compression, ni les outils de compression ne sont soumis à une étape de chauffage avant ou pendant l'étape de compression proprement dite.

3. Comprimés matriciels selon l'une quelconque des revendications 1 et 2, **caractérisés en ce que** le ou lesdits principes actifs sont choisis dans le groupe comprenant les psychotropes, les neuroleptiques, les tranquillisants, les hyptoniques, les analgésiques et les anxiolytiques.

4. Comprimés matriciels selon l'une quelconque des revendications 1 à 3, **caractérisés en ce que** lesdits principes actifs appartiennent au groupe comprenant la morphine, l'oxycodone, l'hydrocodone, l'hydromorphone, l'oxymorphone, le tramadol, la méthadone, la codéine, le fentanyle et la buprénorphine, leurs sels et leurs dérivés pharmaceutiquement acceptables.

5. Comprimés matriciels selon l'une quelconque des revendications 1 à 4, **caractérisés en ce que** ladite matrice de compression comprend en outre au moins un excipient pharmaceutiquement acceptable choisi dans le groupe comprenant les agents anti-collage, les agents capables d'améliorer la cohésion des comprimés lors de la compression, les agents de charge, les lubrifiants, les plastifiants, les agents de remplissage, les édulcorants et les colorants.

6. Comprimés matriciels selon la revendication 5, **caractérisés en ce que** ledit excipient pharmaceutique acceptable représente de 0,1 à 10 % en poids du poids total de la matrice de compression, préférentiellement de 0,5 à 5 % en poids.

7. Comprimés matriciels selon l'une quelconque des revendications 1 à 6, **caractérisés en ce que** ladite matrice de compression comprend en outre au moins une ou plusieurs des substances (a) à (f) qui suivent ou leur mélange :

   a) une substance qui irrite les voies nasale et/ou pharyngée,
   b) un agent augmentant la viscosité qui permet la formation d'un gel lorsque le comprimé est dissous dans un minimum d'eau,

c) une substance émétique,
d) un colorant aversif,
e) une substance au goût amer,
f) un antagoniste du ou des principe(s) actif(s) susceptible(s) d'être détourné(s) à des fins de toxicomanie.

**8.** Comprimés matriciels selon la revendication 7, **caractérisés en ce que** l'agent antagoniste dudit ou desdits principes actifs susceptibles d'être détournés à des fins de toxicomanie est la naloxone ou la naltrexone ou un de leurs sels pharmaceutiquement acceptable.

**9.** Comprimés matriciels selon l'une quelconque des revendications 1 à 8 **caractérisé en ce qu'**il comporte un enrobage externe.

**10.** Comprimés matriciels selon la revendication 9, **caractérisés en ce que** ledit enrobage externe est constitué d'au moins un polymère retard avantageusement choisi dans le groupe comprenant les dérivés d'éthylcellulose et les polymères méthacryliques.

**11.** Comprimés matriciels selon l'une quelconque des revendications 1 à 10, **caractérisés en ce que** ladite matrice est constituée d'un mélange de cellulose microcristalline et [d'acétate de polyvinyle/polyvinylpyrrolidone (80:20)] en proportion (1 :1) et que lesdits principes actifs susceptibles d'être détournés à des fins de toxicomanie appartiennent au groupe des analgésiques.

**12.** Comprimés matriciels selon la revendication 11, **caractérisés en ce qu'**ils comportent un enrobage externe constitué d'éthylcellulose.

**13.** Comprimés matriciels selon l'une quelconque des revendications 1 à 12, **caractérisés en ce qu'**ils sont capables de libérer le principe actif susceptible d'être détourné à des fins de toxicomanie pendant une période supérieure à 12 heures.

**14.** Comprimés matriciels selon l'une quelconque des revendications 1 à 13, **caractérisés en ce qu'**ils sont capables de libérer le principe actif susceptible d'être détourné à des fins de toxicomanie pendant une période supérieure à 20 heures.

**15.** Comprimés matriciels à base d'oxycodone selon la revendication 11, **caractérisés en ce qu'**ils présentent un profil plasmatique après administration unique chez l'être humain, tel que le ratio du Cmax observé après administration desdits comprimés sur le Cmax observé après administration de comprimés d'oxycontin® à libération prolongée (« *Oxycontin® extended release)* de même dosage ne dépasse pas 0,7.

**16.** Comprimés matriciels à base d'oxycodone selon l'une quelconque des revendications 1 à 15, **caractérisés en ce qu'**ils présentent un profil plasmatique après administration unique chez l'être humain, tel que le ratio de l'AUC∞ observée pour lesdits comprimés sur l'AUC∞ observée pour des comprimés d'oxycontin® à libération prolongée (« *Oxycontin® extended release)* de même dosage est compris dans l'intervalle de 80 à 125 %.

**17.** Comprimés selon l'une quelconque des revendications 1 à 16, **caractérisés en ce qu'**ils sont susceptibles d'être administrées une fois par jour.

**18.** Procédé de fabrication des comprimés matriciels selon l'une quelconque des revendications 1 à 16, **caractérisé en ce qu'**il comporte les étapes suivantes :

- mélange du ou des principes actifs avec le ou les excipients de la matrice de compression,
- éventuellement granulation et
- compression dudit mélange,

les forces de compression appliquées étant comprises entre 10 kN et 160 kN.

**19.** Procédé selon la revendication 18, **caractérisé en ce que** l'étape de compression est réalisée sans que le mélange pour compression ou les outils de compression ne soient soumis à une étape de chauffage avant ou pendant l'étape de compression proprement dite.

**20.** Procédé selon l'une quelconque des revendications 18 et 19, **caractérisé en ce qu'**il comporte en outre une étape d'enrobage dudit comprimé matriciel.

21. Procédé selon la revendication 20 **caractérisé en ce qu'**il comporte en outre une étape de maturation dudit enrobage externe.

22. Composition pharmaceutique sous la forme de comprimés selon l'une quelconque des revendications 1 à 17 pour son utilisation en tant que médicament.

23. Comprimés matriciels insolubles dans l'eau capables de libérer l'oxycodone de façon prolongée dans le temps et comprenant 19,83 % en poids du poids total du comprimé, d'oxycodone dispersés au sein d'une matrice de compression, ladite matrice étant constituée de :

- 99.13 % d'un mélange de cellulose microcristalline et [d'acétate de polyvinyle/polyvinylpyrrolidone (80:20)], en poids du poids total de la matrice,
- 0.25 % de silice précipitée, en poids du poids total de la matrice, et
- 0,62 % de stéarate de magnésium, en poids du poids total de la matrice,

**caractérisés en ce que** ladite matrice de compression représente 80,17 % en poids du poids total du comprimé, et **caractérisés en ce que** lesdits comprimés sont obtenus par application d'une force de compression de 35 kN.

**Patentansprüche**

1. Wasserunlösliche Matrixtabletten, die imstande sind, einen oder mehrere Wirkstoffe im Organismus über einen längeren Zeitraum abzugeben und mindestens einen in einer Kompressionsmatrix verteilten Wirkstoff enthalten, der zum Zweck der Drogensucht missbrauchbar ist, wobei die Matrix von einem Gemisch aus mikrokristalliner Zellulose und [Polyvinyl-/Polyvinylpyrrolidonacetat (80:20)] gebildet ist, **dadurch gekennzeichnet, dass**:

- der Wirkstoff 10 bis 50 Gew.-% des Gesamtgewichts der Tablette darstellt,
- die Kompressionsmatrix 50 bis 98 Gew.-%, in vorteilhafter Weise 85 bis 95 Gew.-%, des Gesamtgewichts der Tablette darstellt,
- das Gemisch aus mikrokristalliner Zellulose und [Polyvinyl-/Polyvinylpyrrolidonacetat (80:20)] 40 bis 100 Gew.-%, in vorteilhafter Weise 50 bis 90 Gew.-%, des Gesamtgewichts der Matrix darstellt, und erhalten werden durch Anwendung von Kompressionskräften von 10 kN bis 160 kN.

2. Matrixtabletten nach Anspruch 1, **dadurch gekennzeichnet, dass** weder das zu komprimierende Gemisch noch die Kompressionswerkzeuge einem Erhitzungsschritt vor oder während des eigentlichen Kompressionsschritts unterzogen werden.

3. Matrixtabletten nach einem der Ansprüche 1 und 2, **dadurch gekennzeichnet, dass** der oder die Wirkstoffe aus der Gruppe ausgewählt ist/sind, die die Psychotropika, die Neuroleptika, die Beruhigungsmittel, die Hypnotika, die Analgetika und die Anxiolytika umfasst.

4. Matrixtabletten nach einem der Ansprüche 1 bis 3, **dadurch gekennzeichnet, dass** die Wirkstoffe zu der Gruppe gehören, die das Morphin, das Oxycodon, das Hydrocodon, das Hydromorphon, das Oxymorphon, das Tramadol, das Methadon, das Kodein, das Fentanyl und das Buprenorphin, ihre Salze und ihre pharmazeutisch akzeptablen Derivate umfasst.

5. Matrixtabletten nach einem der Ansprüche 1 bis 4, **dadurch gekennzeichnet, dass** die Kompressionsmatrix ferner mindestens einen pharmazeutisch akzeptablen Arzneiträger umfasst, der aus der Gruppe ausgewählt ist, die die Anti-Klebemittel, die Mittel, die imstande sind, die Kohäsion der Tabletten bei der Kompression zu verbessern, die Chargenmittel, die Gleitmittel, die Weichmacher, die Füllstoffe, die Süßstoffe und die Farbstoffe umfasst.

6. Matrixtabletten nach Anspruch 5, **dadurch gekennzeichnet, dass** der pharmazeutisch akzeptable Arzneiträger 0,1 bis 10 Gew.-%, vorzugsweise 0,5 bis 5 Gew.-%, des Gesamtgewichts der Kompressionsmatrix darstellt.

7. Matrixtabletten nach einem der Ansprüche 1 bis 6, **dadurch gekennzeichnet, dass** die Kompressionsmatrix ferner mindestens eine oder mehrere der folgenden Substanzen (a) bis (f) oder ihr Gemisch umfasst:

a) eine Substanz, die den Nasen- und/oder Rachenweg reizt,
b) ein Mittel, das die Viskosität erhöht, was die Bildung eines Gels erlaubt, wenn die Tablette in einem Minimum Wasser aufgelöst wird,
c) eine emetische Substanz,
d) einen Abneigung hervorrufenden Farbstoff,
e) eine bitter schmeckende Substanz,
f) einen Antagonisten des Wirkstoffs oder der Wirkstoffe, der/die zu Zwecken der Drogensucht missbraucht werden könnte/n.

**8.** Matrixtabletten nach Anspruch 7, **dadurch gekennzeichnet, dass** das antagonistische Mittel des Wirkstoffs oder der Wirkstoffe, der/die zu Zwecken der Drogensucht missbrauchbar ist/sind, das Naloxon oder das Naltrexon oder eines ihrer pharmazeutisch akzeptablen Salze ist.

**9.** Matrixtabletten nach einem der Ansprüche 1 bis 8, **dadurch gekennzeichnet, dass** sie eine äußere Umhüllung aufweisen.

**10.** Matrixtabletten nach Anspruch 9, **dadurch gekennzeichnet, dass** die äußere Umhüllung von mindestens einem Retard-Polymer gebildet ist, das in vorteilhafter Weise aus der Gruppe ausgewählt ist, die die Ethylcellulosederivate und die Methacrylpolymere umfasst.

**11.** Matrixtabletten nach einem der Ansprüche 1 bis 10, **dadurch gekennzeichnet, dass** die Matrix von einem Gemisch aus mikrokristalliner Zellulose und [Polyvinyl-/Polyvinylpyrrolidonacetat (80:20)] im Verhältnis (1:1) gebildet ist und dass die Wirkstoffe, die zum Zweck der Drogensucht missbrauchbar sind, zur Gruppe der Analgetika gehören.

**12.** Matrixtabletten nach Anspruch 11, **dadurch gekennzeichnet, dass** sie eine äußere Umhüllung aufweisen, die aus Ethylcellulose besteht.

**13.** Matrixtabletten nach einem der Ansprüche 1 bis 12, **dadurch gekennzeichnet, dass** sie imstande sind, den Wirkstoff, der zum Zweck der Drogensucht missbrauchbar ist, über einen Zeitraum von mehr als 12 Stunden freizusetzen.

**14.** Matrixtabletten nach einem der Ansprüche 1 bis 13, **dadurch gekennzeichnet, dass** sie imstande sind, den Wirkstoff, der zum Zweck der Drogensucht missbrauchbar ist, über einen Zeitraum von mehr als 20 Stunden freizusetzen.

**15.** Matrixtabletten auf der Basis von Oxycodon nach Anspruch 11, **dadurch gekennzeichnet, dass** sie ein plasmatisches Profil nach einziger Verabreichung beim Menschen aufweisen, so dass das Verhältnis des Cmax, beobachtet nach Verabreichung der Tabletten, zum Cmax, beobachtet nach Verabreichung von Oxycontin®-Tabletten mit längerer Freisetzung (*"Oxycontin® extended release")* mit derselben Dosierung, 0,7 nicht überschreitet.

**16.** Matrixtabletten auf der Basis von Oxycodon nach einem der Ansprüche 1 bis 15, **dadurch gekennzeichnet, dass** sie ein plasmatisches Profil nach einziger Verabreichung beim Menschen aufweisen, so dass das Verhältnis von $AUC_\infty$, beobachtet für die Tabletten, zu $AUC_\infty$, beobachtet für Oxycontin®-Tabletten mit längerer Freisetzung (*"Oxycontin® extended release"*) mit derselben Dosierung, im Intervall von 80 bis 125 % liegt.

**17.** Tabletten nach einem der Ansprüche 1 bis 16, **dadurch gekennzeichnet, dass** sie einmal täglich verabreichbar sind.

**18.** Verfahren zur Herstellung der Matrixtabletten nach einem der Ansprüche 1 bis 16, **dadurch gekennzeichnet, dass** es die folgenden Schritte aufweist:

- Mischen des oder der Wirkstoffe mit dem oder den Arzneiträgern der Kompressionsmatrix,
- eventuell Granulieren und
- Komprimieren des Gemischs,

wobei die angewendeten Kompressionskräfte 10 kN bis 160 kN betragen.

**19.** Verfahren nach Anspruch 18, **dadurch gekennzeichnet, dass** der Kompressionsschritt durchgeführt wird, ohne dass das zu komprimierende Gemisch oder die Kompressionswerkzeuge einem Erhitzungsschritt vor oder während des eigentlichen Kompressionsschritts unterzogen werden.

**20.** Verfahren nach einem der Ansprüche 18 und 19, **dadurch gekennzeichnet, dass** es ferner einen Umhüllungsschritt der Matrixtablette aufweist.

**21.** Verfahren nach Anspruch 20, **dadurch gekennzeichnet, dass** es ferner einen Reifeschritt der äußeren Umhüllung aufweist.

**22.** Pharmazeutische Zusammensetzung in Form von Tabletten nach einem der Ansprüche 1 bis 17 für ihre Verwendung als Arzneimittel.

**23.** Wasserunlösliche Matrixtabletten, die imstande sind, das Oxycodon über einen längeren Zeitraum abzugeben und die 19,83 Gew.-% des Gesamtgewichts der Tablette von Oxycodon umfassen, das innerhalb einer Kompressionsmatrix verteilt ist, wobei die Matrix gebildet ist von:

- 99,13 Gew.-% des Gesamtgewichts der Matrix eines Gemischs aus mikrokristalliner Zellulose und [Polyvinyl-/Polyvinylpyrrolidonacetat (80:20)],
- 0,25 Gew.-% des Gesamtgewichts der Matrix von ausgefälltem Siliziumoxid, und
- 0,62 Gew.-% des Gesamtgewichts der Matrix von Magnesiumstearat,

**dadurch gekennzeichnet, dass** die Kompressionsmatrix 80,17 Gew.-% des Gesamtgewichts der Tablette darstellt, und **gekennzeichnet dadurch, dass** die Tabletten durch Anwenden einer Kompressionskraft von 35 kN hergestellt werden.

## Claims

**1.** Water-insoluble, matrix tablets capable of releasing one or more active ingredients into the body over an extended time period and containing at least one active ingredient which may be subject to drug abuse dispersed within a compression matrix, said matrix consisting of a mixture of microcrystalline cellulose and [polyvinyl acetate/polyvinylpyrrolidone (80:20)], **characterized in that**:

- said active ingredient represents 10 to 50 weight % of the total weight of the tablet,
- said compression matrix represents 50 to 98 weight % of the total weight of said tablet, advantageously 85 to 95 %,
- said mixture of microcrystalline cellulose and [polyvinyl acetate/polyvinylpyrrolidone (80:20)] represents 40 to 100 %, advantageously 50 to 90 weight % of the total weight of the matrix, and obtained by applying compression forces comprised between 10 kN and 160 kN.

**2.** Matrix tablets according to claim 1, **characterized in that** neither the mixture to be compressed, nor the compression tooling are subjected to a heating step either before or during the actual compression step.

**3.** Matrix tablets according to either of claims 1 and 2, **characterized in that** said active ingredient(s) are chosen from the group comprising psychotropics, neuroleptics, tranquillizers, hypnotics, analgesics and anxiolytics.

**4.** Matrix tablets according to any of claims 1 to 3, **characterized in that** said active ingredients belong to the group comprising morphine, oxycodone, hydrocodone, hydromorphone, oxymorphone, tramadol, methadone, codeine, fentanyl and buprenorphine, their salts and their pharmaceutically acceptable derivatives.

**5.** Matrix tablets according to any of claims 1 to 4, **characterized in that** said compression matrix also contains at least one pharmaceutically acceptable excipient chosen from the group comprising anti-adherent agents, agents capable of improving tablet cohesion on compressing, fillers, lubricants, plasticizers, bulking agents, sweeteners and colouring agents.

**6.** Matrix tablets according to claim 5, **characterized in that** said pharmaceutically acceptable excipient represents 0.1 to 10 weight % of the total weight of the compression matrix, preferably between 0.5 and 5 weight %.

**7.** Matrix tablets according to any of claims 1 to 6, **characterized in that** said compression matrix also contains at least one or more of the following substances (a) to (f) or a mixture thereof:

a) a substance which irritates the nasal and/or pharyngeal tracts,
b) a viscosity-increasing agent, leading to formation of a gel when the tablet is dissolved in a minimum amount of water,
c) an emetic substance,
d) an aversive colouring agent,
e) a bittering substance,
f) an antagonist of the active ingredient(s) which may be the subject of drug abuse.

**8.** Matrix tablets according to claim 7, **characterized in that** the antagonist agent of said active ingredient(s) which may be the subject of drug abuse is naloxone or naltrexone or one of their pharmaceutically acceptable salts.

**9.** Matrix tablets according to any of claims 1 to 8, **characterized in that** they have an outer coating.

**10.** Matrix tablets according to claim 9, **characterized in that** said outer coating consists of at least one sustained-release polymer advantageously chosen from the group comprising ethylcellulose derivatives and methacrylic polymers.

**11.** Matrix tablets according to any of claims 1 to 10, **characterized in that** said matrix consists of a mixture of microcrystalline cellulose and [polyvinyl acetate/polyvinylpyrrolidone (80:20)] to the proportion of (1:1) and that said active ingredients which may be the subject of drug abuse belong to the group of analgesics.

**12.** Matrix tablets according to claim 11, **characterized in that** they have an outer coating consisting of ethylcellulose.

**13.** Matrix tablets according to any of claims 1 to 12, **characterized in that** they are capable of releasing the active ingredient which may be subject to drug abuse over a period of more than 12 hours.

**14.** Matrix tablets according to any of claims 1 to 13, **characterized in that** they are capable of releasing the active ingredient which may be subject to drug abuse over a period of more than 20 hours.

**15.** Matrix tablets containing oxycodone according to claim 11, **characterized in that** they have a plasma profile after once-$\alpha$-day administration in man, such that the ratio of the Cmax observed after administration of said tablets to the Cmax value observed after administration of Oxycontin® extended release tablets containing the same dose, does not exceed 0.7.

**16.** Matrix tablets containing oxycodone according to any of claims 1 to 15, **characterized in that** their plasma profile after once-$\alpha$-day administration in man is such that the ratio of $AUC\infty$ observed for said tablets to the $AUC\infty$ value observed with Oxycontin® extended release tablets containing the same dose, lies in the interval of 80 to 125 %.

**17.** Tablets according to any of claims 1 to 16, **characterized in that** they can be administered once a day.

**18.** Method to produce matrix tablets according to any of claims 1 to 16, **characterized in that** it comprises the following steps:

- mixing the active ingredient(s) with the excipient(s) of the compression matrix,
- optional granulation, and
- compressing said mixture,

the applied compression forces lie between 10 kN and 160 kN.

**19.** Method according to claim 18, **characterized in that** the compression step is conducted without the compression mixture or the compression tooling being subjected to a heating step either before or during the actual compression step.

**20.** Method according to either of claims 18 and 19, **characterized in that** it also comprises a coating step of said matrix tablet.

**21.** Method according to claim 20, **characterized in that** it also comprises a curing step of said outer coating.

**22.** Pharmaceutical composition in the form of tablets according to any of claims 1 to 17, for use as a drug.

**23.** Water-insoluble, matrix tablets capable of releasing oxycodone over an extended time period and containing 19.83 weight % of the total weight of the tablet of oxycodone within a compression matrix, said matrix consisting of:

- 99.13 weight % of a mixture of microcrystalline cellulose and [polyvinyl acetate/polyvinylpyrrolidone (80:20)] of the total weight of the matrix,
- 0.25 weight % of precipitated silica of the total weight of the matrix, and
- 0.62 weight % of magnesium stearate of the total weight of the matrix,

**characterized in that** said compression matrix represents 80.17 weight % of the total weight of the tablet, and **characterized in that** said tablets are obtained by applying a compression force of 35 kN.

**Figure 1**

Profils de dissolution à pH 6,8 de comprimés d'oxycodone HCl à 40mg non pelliculés, obtenus selon l'exemple 1,

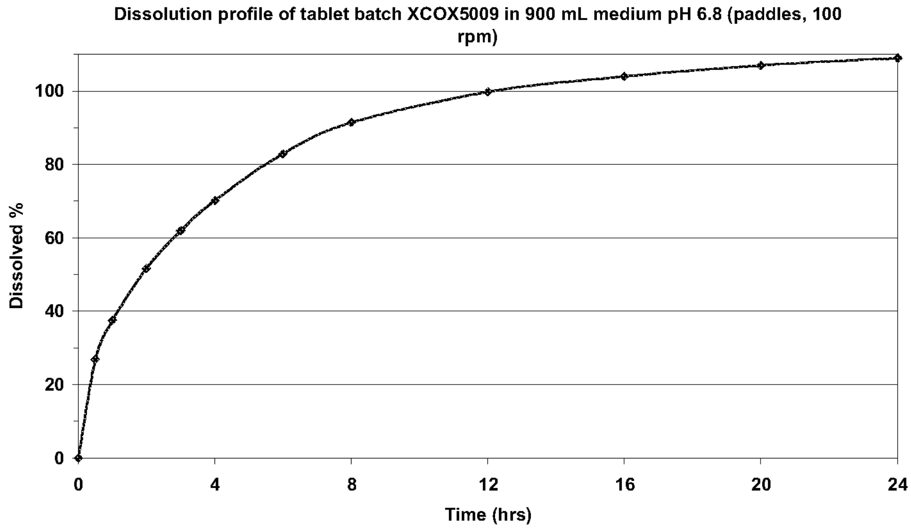


**Figure 2**

Profils de dissolution à pH 6,8 de comprimés d'oxycodone HCl à 40mg non pelliculés, obtenus selon l'exemple 2,

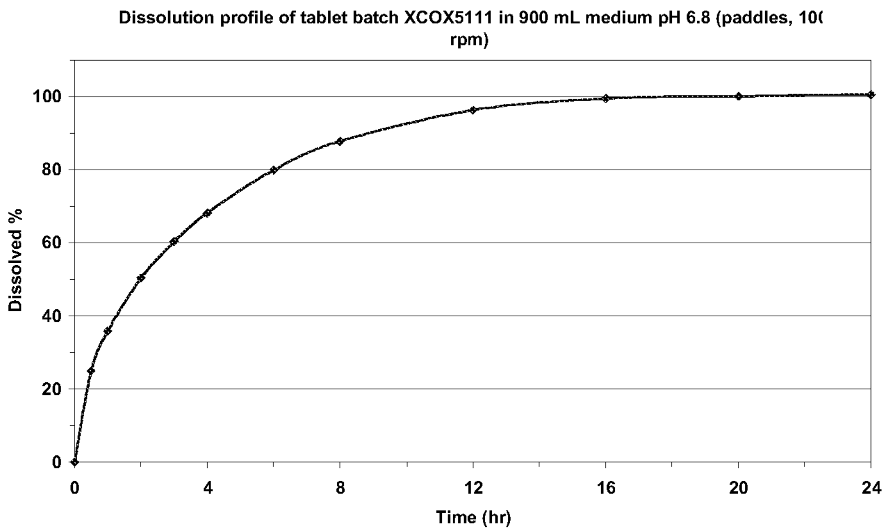

**Figure 3**

Profil de dissolution à pH 6,8 de comprimés conformes à l'exemple 2, pelliculés avec une couche d'Ethylcellulose EC30 D et ayant subi une étape de maturation

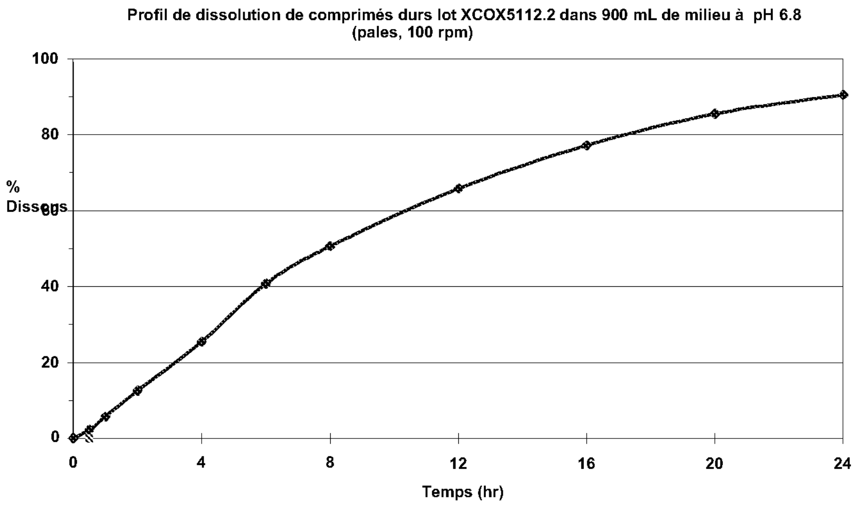

**Figure 4**

Profils de dissolution comparatifs de comprimés matriciels d'oxycodone conformes à l'invention dans un milieu HCl 0,1 N dépourvu d'éthanol et dans un milieu HCl 0,1 N comprenant 40 % d'éthanol

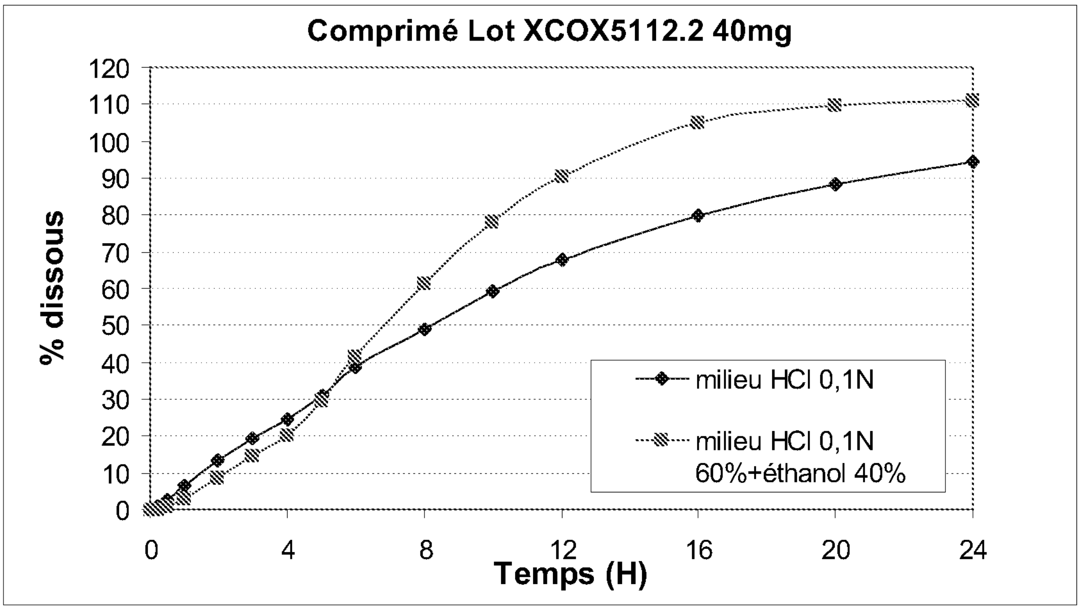

**Figure 5**

profils de dissolution de comprimés matriciels d'oxycodone conformes à l'invention dans deux milieux de dissolution de pH différents (1,2 et 6,8)

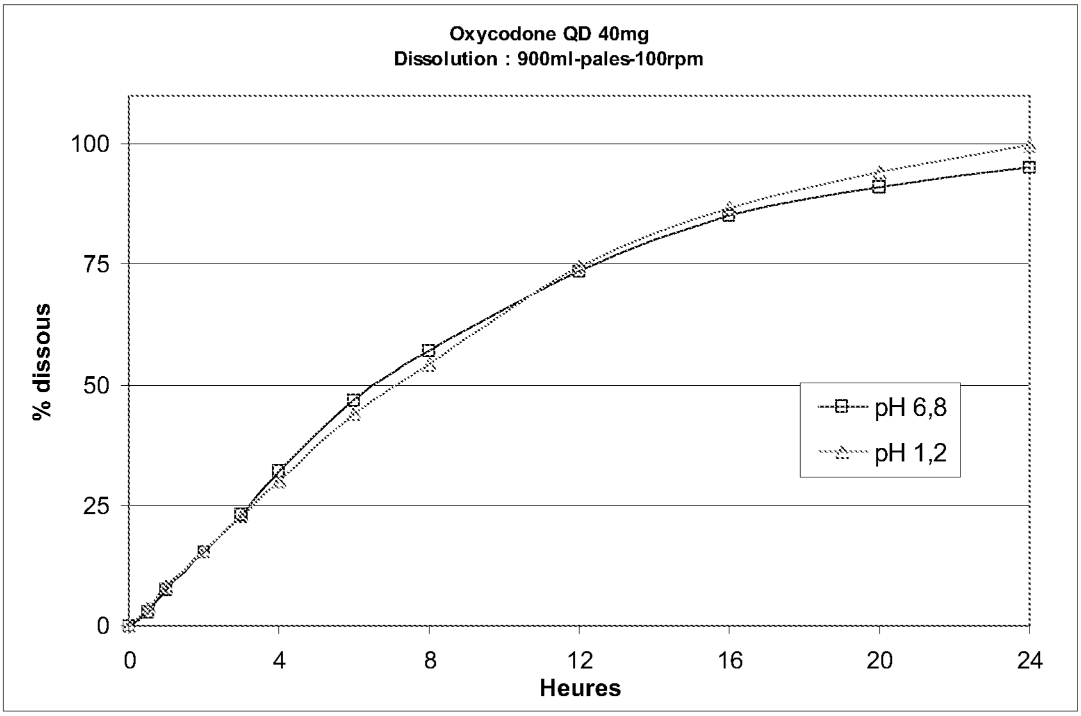

# Figure 6

Profils de dissolution sur 24 heures de comprimés d'oxycodone dosés à 40 mg conformes à l'invention après une période de stockage en blisters alu/alu en conditions de stabilité accélérée de 1 mois, 2 mois, 3 mois et 6 mois.

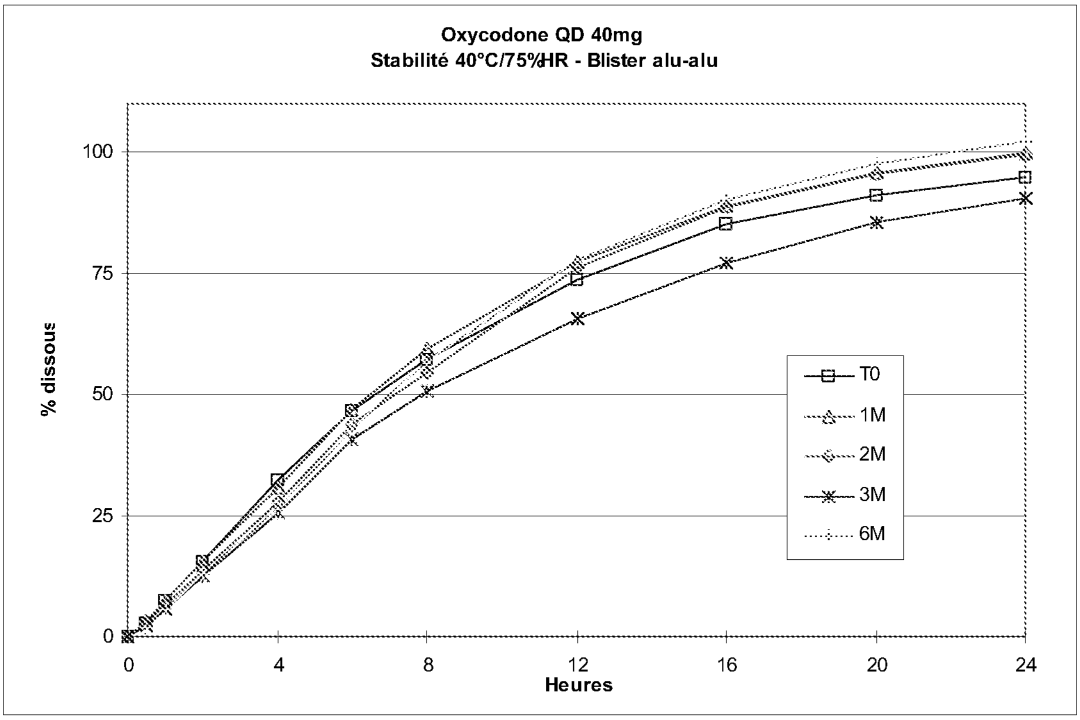

**Figure 7**

Profils de dissolution sur 24 heures de comprimés d'oxycodone dosés à 20 mg conformes à l'invention après une période de stockage en bouteilles HDPE avec un déshydratant en conditions de stabilité accélérée de 1 mois, 2 mois et 3 mois.

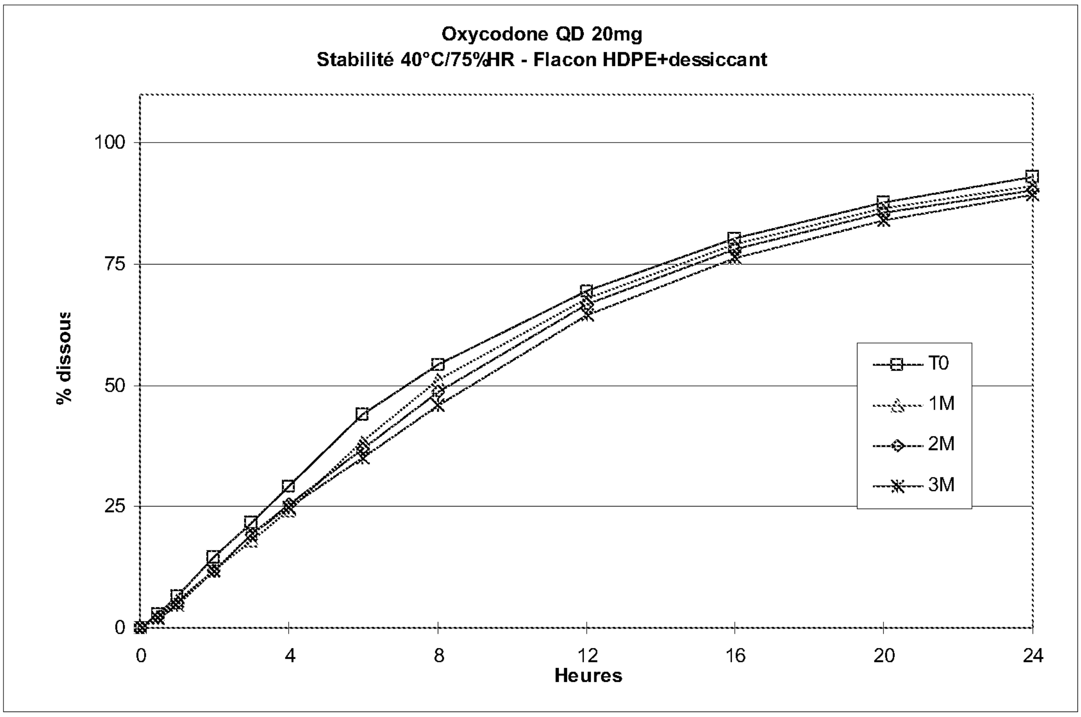

**Figure 8**

Profils plasmatiques d'oxycodone après administration unique de comprimés d'oxycodone dosés à 40 mg conformes à l'invention et de comprimés d'oxycodone du produit de référence Oxycontin®, dosés à 40 mg

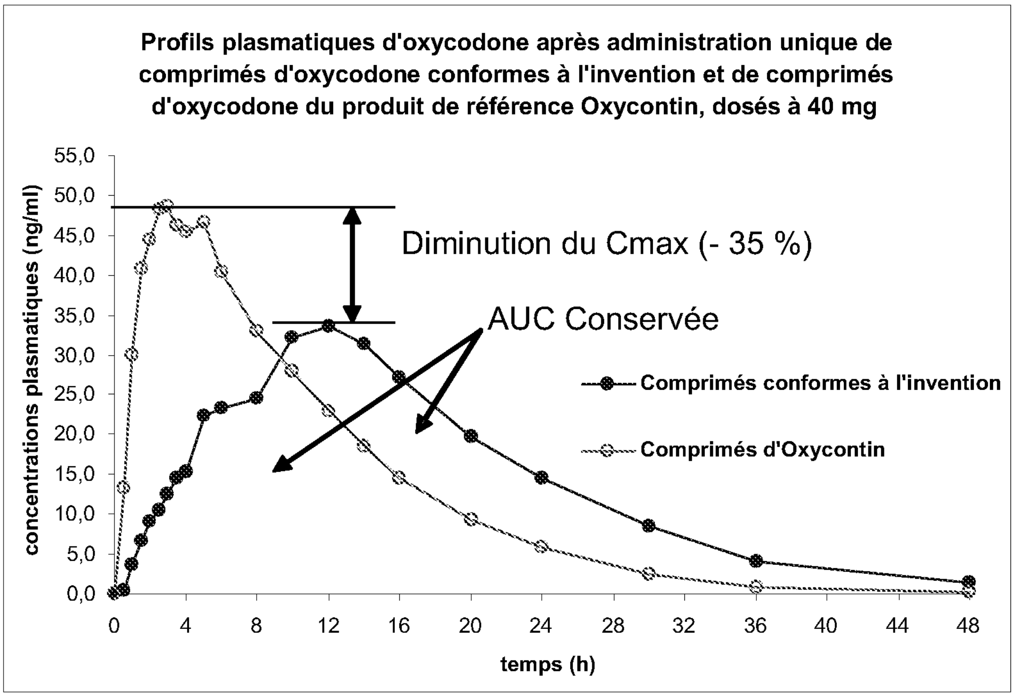

**Figure 9**

Profil de dissolution sur 24 heures de comprimés non enrobés ultra-durs d'oxycodone et de naloxone à pH 6,8.

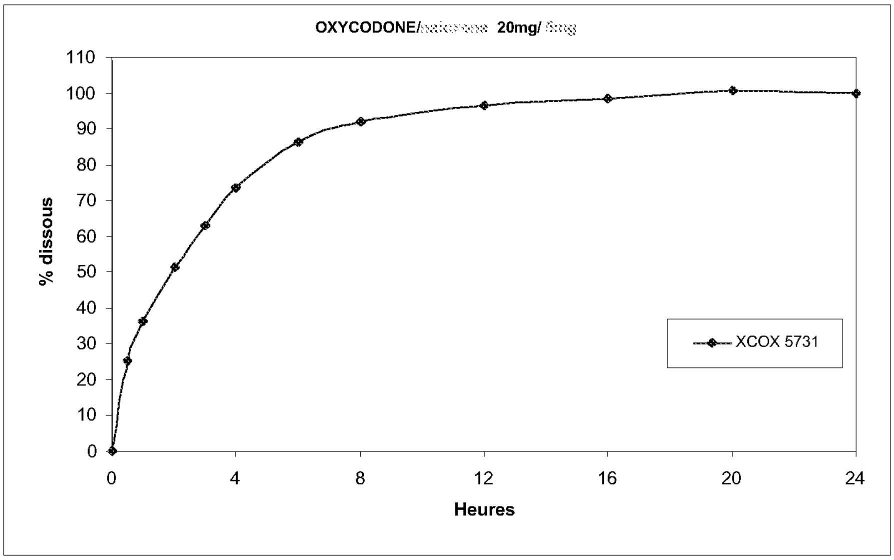


**Figure 10**

Profils de dissolution sur 10 heures de comprimés non enrobés ultra-durs d'oxycodone comprenant une matrice à base d'excipients minéraux dosés à 20 mg à pH 6,8.

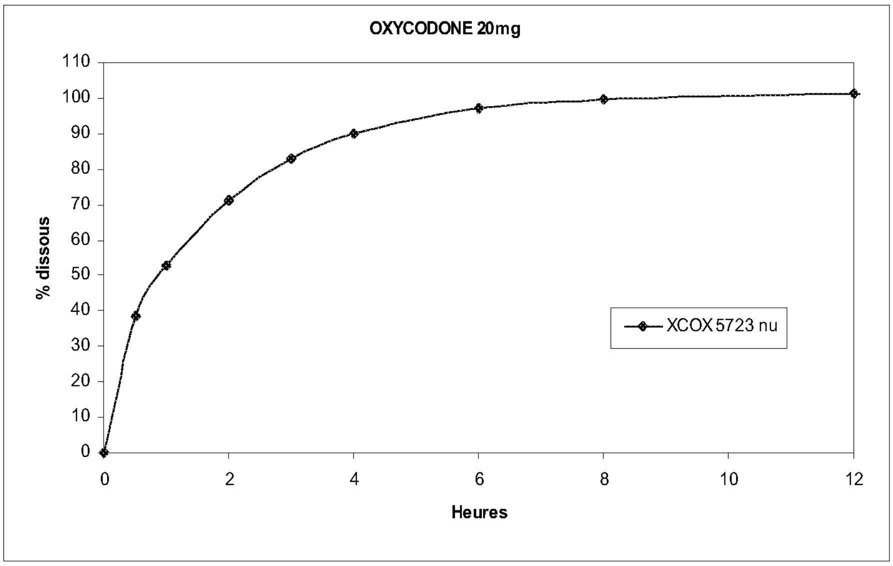

**Figure 11**

Profils de dissolution de comprimés conformes à l'invention (« QD ») et de comprimés du produit de référence Oxycontin® (réf) à pH 6,8, pour des comprimés entiers, coupés en deux ou broyés (« en morceaux »)

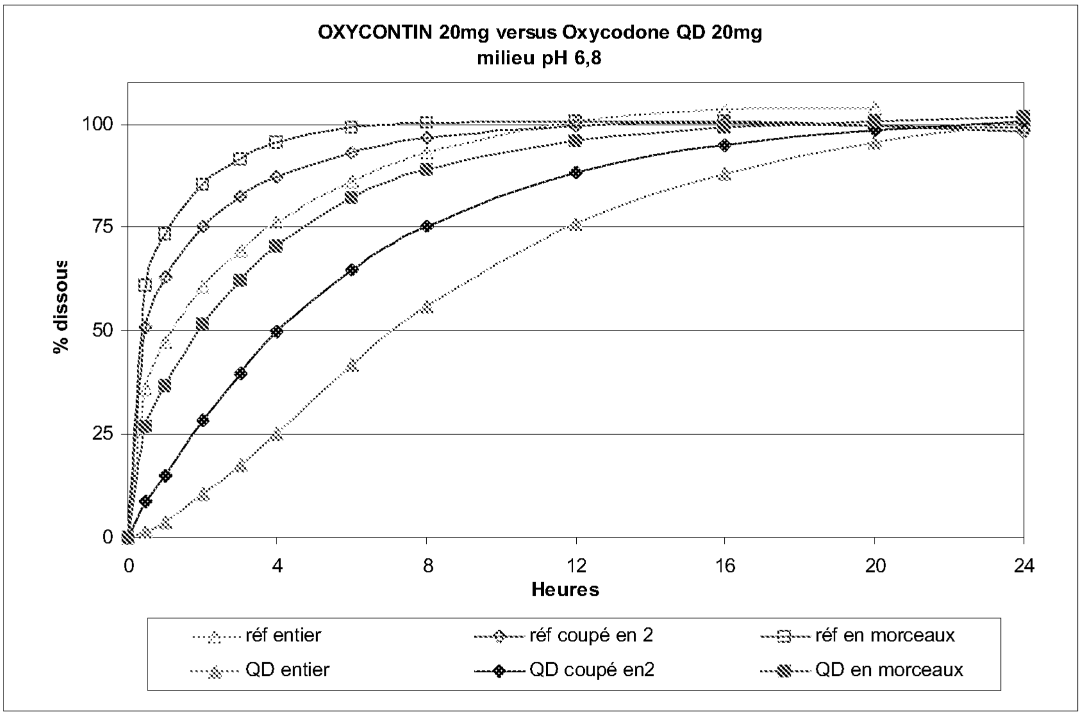

**Figure 12**

Profil de dissolution de microgranules d'hydromorphone en milieu aqueux (HCl 0,1 N) dans trois conditions : sans éthanol, en présence de 20% d'éthanol et en présence de 40% d'éthanol pendant 4 heures

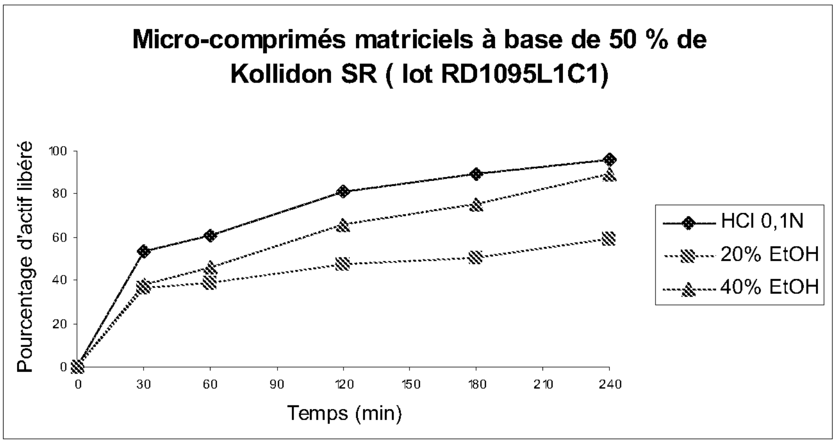

## Figure 13

Profil de dissolution de microgranules de sulfate de morphine dans un milieu de dissolution à pH 6,8 sans éthanol, à pH 6,8 en présence de 20% d'éthanol et à pH 6,8 en présence de 40% d'éthanol pendant 12 heures

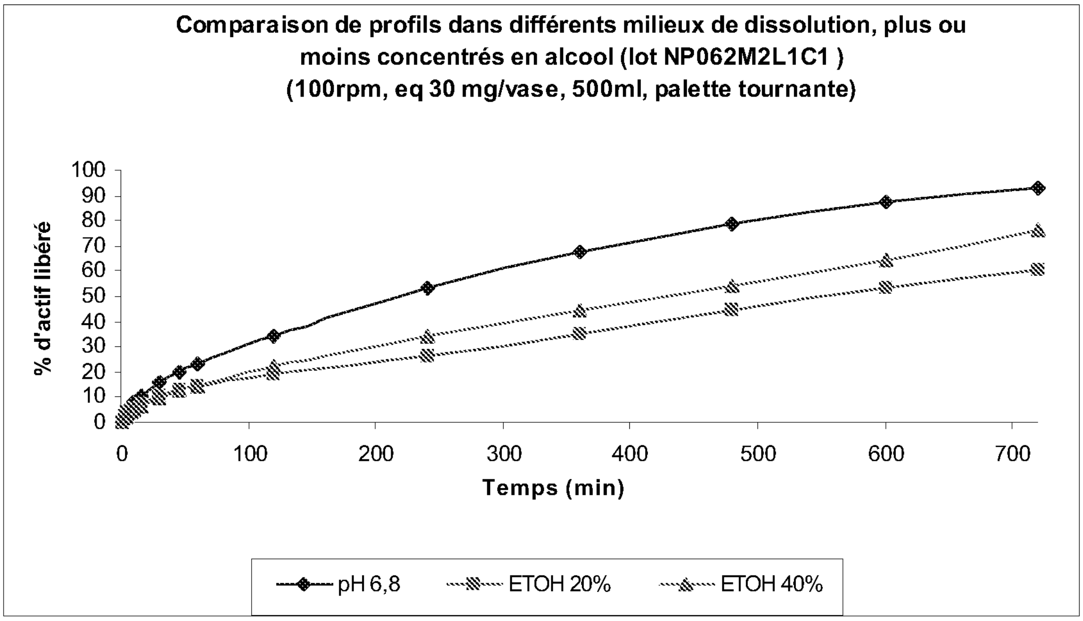

**Figure 14**

Tests de dissolution comparatifs entre des comprimés ultra-durs de sulfate de morphine conformes à l'invention et des comprimés de sulfate de morphine du commerce (Avinza®) A) 20% d'éthanol dans un milieu HCl 0,1 N ; B) 40% éthanol dans un milieu HCl 0,1 N

A)

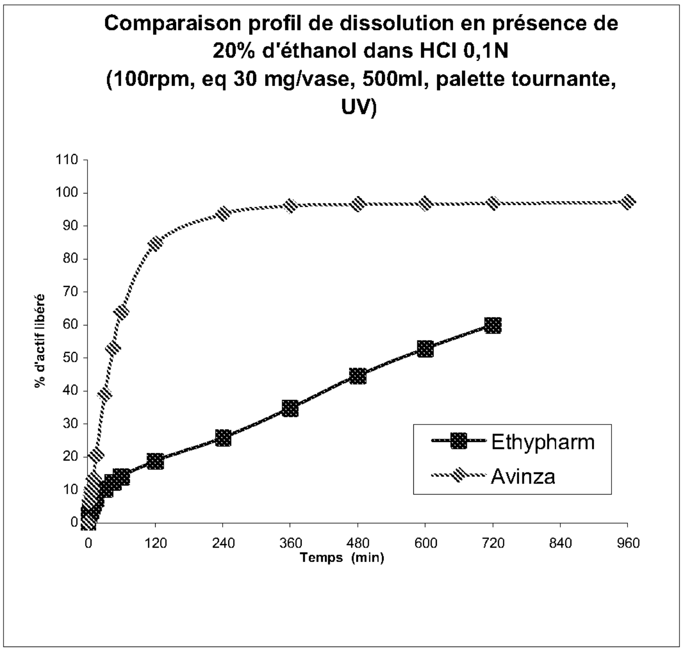


B)

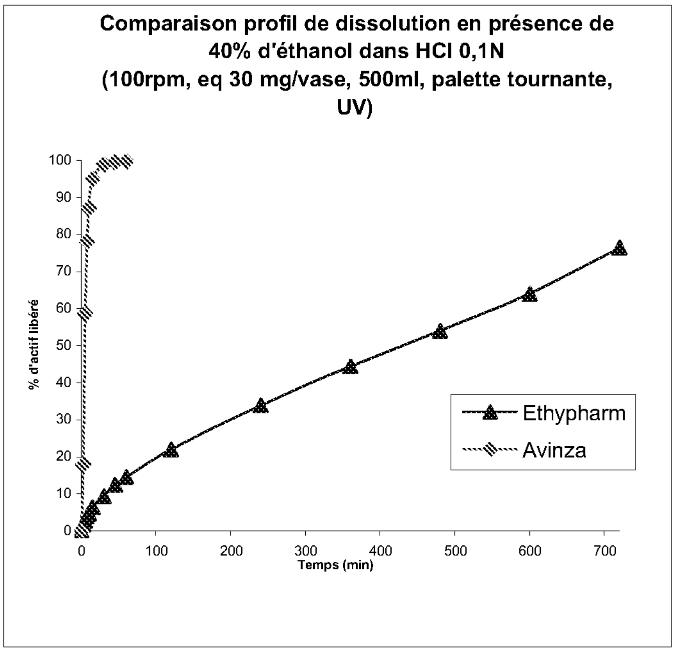

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 3966940 A
- US 3773955 A
- EP 0185472 A
- EP 0319243 A
- US 20030143269 A
- US 20030068392 A
- US 20050281748 A
- US 20030118641 A
- US 5747058 A
- US 6413536 B
- EP 0974355 A
- EP 0933079 A
- EP 0997143 A
- US 6592901 B
- US 20050031546 A


### Littérature non-brevet citée dans la description


- **PONTIER et al.** *Journal of European Ceramic Society,* 2002, vol. 22
- **C. PONTIER.** Les phosphates de calcium apatitiques en compression. De la chimie aux qualités d'usage. *Thèse de l'Université de Paris XI,* 25 Septembre 2001
- **FELL, J.T. ; NEWTON, J.M.** Détermination of tablet strength by the diametral-compression test. *J. Pharm. Sci.,* 1970, vol. 59 (5), 688-691